(19) Europäisches Patentamt
European Patent Office
Office européen des brevets

(11) Veröffentlichungsnummer: **0 033 384**
**B1**

(12) # EUROPÄISCHE PATENTSCHRIFT

(45) Veröffentlichungstag der Patentschrift: **15.02.84**

(51) Int. Cl.³: **C 07 C 103/52,** A 61 K 37/02

(21) Anmeldenummer: **80107997.1**

(22) Anmeldetag: **17.12.80**

(54) **Thymosin-alpha-1-Fragmente enthaltende Arzneimittel mit immunstimulierender Wirkung, und Thymosin-alpha-1-Fragmente.**

(30) Priorität: **18.01.80 DE 3001775**
**25.03.80 US 133708**

(43) Veröffentlichungstag der Anmeldung: **12.08.81 Patentblatt 81/32**

(45) Bekanntmachung des Hinweises auf die Patenterteilung: **15.02.84 Patentblatt 84/7**

(84) Benannte Vertragsstaaten: **BE CH DE FR GB LI NL**

(56) Entgegenhaltungen:
**EP - A - 0 026 464**
**WO - A - 80/02560**
**DE - A - 2 817 082**
**US - A - 4 079 127**

**ANGEWANDTE CHEMIE, International Edition in English, 18th May 1979, Seiten 394-395, Verlag Chemie GmbH Weinheim, DE. C. BIRR et al.: "Synthesis of Thymosin alpha 1, a Polypeptide of the Thymus"**

(73) Patentinhaber: **Max-Planck-Gesellschaft zur Förderung der Wissenschaften e.V., Bunsenstrasse 10, D-3400 Göttingen (DE)**

(72) Erfinder: **Birr, Christian, Dr., Eichendorffstrasse 31, D-6906 Leimen/St.Ilgen (DE)**
Erfinder: **Stollenwerk, Ulrich, *, verstorben (DE)**
Erfinder: **Werner, Ilona, Görresstrasse 38, D-6900 Heidelberg (DE)**

(74) Vertreter: **Weickmann, Heinrich, Dipl.-Ing. et al, Patentanwälte Dipl.-Ing. H. Weickmann Dipl.-Phys.Dr. K. Fincke Dipl.-Ing. F.A. Weickmann Dipl.-Chem. B. Huber Dr.-Ing. H. Liska Möhlstrasse 22, D-8000 München 86 (DE)**

EP 0 033 384 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Thymosin$\alpha_1$-Fragmente enthaltende Arzneimittel mit immunstimulierender Wirkung, und Thymosin$\alpha_1$-Fragmente

Die Erfindung betrifft Arzneimittel mit immunstimulierender bzw. immunregulierender Wirkung, das zur Behandlung von Immunmangelerkrankungen, von Virusinfektionen, des beschleunigten Alterns, Tumorbildungswahrscheinlichkeit und insbesondere von Krebs eingesetzt werden kann, sowie Thymosin$\alpha_1$-Fragmente, die in diesem Arzneimittel als Wirkstoff enthalten sind.

Die Beeinflussung des Immunapparats des Körpers durch Polypeptide der Thymusdrüse wird in jüngster Zeit mit grossem Interesse und zunehmendem Erfolg untersucht. Dies ist eine Folge der Erkenntnis, dass zellfreie Proteinextrakte der Thymusdrüse von Kälbern, wie das Standardpräparat der Bezeichnung Thymosinfraktion Nr. 5, in unterschiedlichem Ausmass Immunmangelerscheinungen unterdrücken, wie eine verminderte Abstossung von Transplantaten, eine zunehmende Infektionsempfindlichkeit, beschleunigtes Altern und erhöhte Wahrscheinlichkeit des Auftretens von Tumoren. In jüngster Zeit wurde berichtet, dass die klinische Anwendung der Thymosinfraktion Nr. 5 an Patienten, die an Leukämie oder anderen Krebsarten litten, bereits zu Heilungseffekten geführt hat, besonders bei Lungenkrebs (P.B. Chretien et al. J.D. Cancer Treat. Rep. 62 (1978) 1787-1790).

1977 gelang es A.L. Goldstein et al. (J. Proc. Natl. Acad. Sci. USA, 74 (1977) 725) aus der Thymosin-Polypeptidmischung einen sauren Bestandteil in reiner Form abzutrennen, den sie als Thymosin$\alpha_1$ bezeichneten und für den sie auch die Peptidsequenz angaben. Thymosin$\alpha_1$ besitzt mit 28 Aminosäuren ein Molekulargewicht von 3107 und reagiert mit neun sauren Seitenfunktionen und nur vier basischen Gruppen sauer. Thymosin$\alpha_1$ besitzt die in der Fig. 1 dargestellte Aminosäuresequenz und Sekundärstruktur (dabei steht Ac für die Acetylgruppe).

Da sich Thymosin$\alpha_1$ nur sehr mühsam aus Thymusdrüsen isolieren lässt, wurde bereits eine Methode zu seiner Totalsynthese vorgeschlagen (J. A. C. S. 101, 1 (1979) 253-254). Auch die ältere Patentanmeldung P 2 919 592.4 der Anmelderin hat ein solches Verfahren zur Herstellung von Thymosin$\alpha_1$ und Derivaten davon zum Gegenstand.

Es hat sich nunmehr überraschenderweise gezeigt, dass bereits Fragmente des Thymosin$\alpha_1$ die gleichen immunregulierenden oder immunstimulierenden Wirkungen ausüben, wie Thymosin$\alpha_1$, wenngleich auch in geringerem Ausmass. So lassen sich bestimmte Thymosin$\alpha_1$-Fragmente, die Gegenstand der vorliegenden Erfindung sind, zur Behandlung von Immunmangelkrankheiten, wie von T-Zellmangelzuständen, des beschleunigten Alterns, der erhöhten Tumorbildungswahrscheinlichkeit und insbesondere von Krebs einsetzen. Dabei wird der geringfügige Nachteil der etwas schwächeren Wirkung der erfindungsgemässen Thymosin$\alpha_1$-Fragmente gegenüber Thymosin$\alpha_1$ dadurch bei weitem kompensiert, dass sich diese Fragmente wesentlich einfacher, mit höheren Ausbeuten und besserer Reinheit herstellen lassen, wodurch eine klinische Anwendung dieser Thymosin$\alpha_1$-Fragmente in der Krebstherapie möglich wird.

Gegenstand der Erfindung sind daher Arzneimittel mit immunstimulierender Wirkung, die als Wirkstoff mindestens ein Thymosin$\alpha_1$-Fragment und/oder mindestens ein Derivat davon in freier Form oder in Form eines pharmakologisch annehmbaren Salzes enthalten, ausgenommen solche der allgemeinen Formel X-Glu-Asn-OH, worin X die Bedeutung H, H-Ala-, H-Glu-Ala-, H-Glu-Glu-Ala-, H-Val-Glu-Glu-Ala- oder H-Val-Val-Glu-Glu-Ala- bedeutet und deren physiologisch verträgliche Salze. Dieses erfindungsgemässe Arzneimittel kann auch zwei oder mehrere der nachstehend definierten Thymosin$\alpha_1$-Fragmente enthalten und enthält neben dem oder den Wirkstoffen übliche, für den gewählten Verabreichungsweg geeignete, pharmakologisch annehmbare Bindemittel, Trägermaterialien und/oder Hilfsstoffe.

Die Thymosin$\alpha_1$-Fragmente, die als Wirkstoffe in dem erfindungsgemässen Arzneimittel enthalten sind und die ebenfalls Gegenstand der vorliegenden Erfindung sind, sind die folgenden:

| | |
|---|---|
| | 1 2 3 4 5 6 |
| Fragment I: | Ser-Asp-Ala-Ala-Val-Asp |
| | 1 2 3 4 5 6 |
| Fragment Ia: | Lys-Asp-Ala-Ala-Val-Asp |
| | 7 8 9 10 11 12 |
| Fragment II: | Thr-Ser-Ser-Glu-Ile-Thr |
| | 13 14 15 16 17 18 19 |
| Fragment III: | Thr-Lys-Asp-Leu-Lys-Glu-Lys |
| | 20 21 22 23 24 |
| Fragment IV: | Lys-Glu-Val-Val-Glu |
| | 20 21 22 23 24 |
| Fragment IVa: | Lys-Glu-Val-Val-Gla |
| | 25 26 27 28 |
| Fragment Va: | Gla-Ala-Glu-Asn |
| | 20 21 22 23 24 25 |
| Fragment VI: | Lys-Glu-Val-Val-Glu-Glu- |
| | 26 27 28 |
| | Ala-Glu-Asn |
| | 20 21 22 23 24 25 |
| Fragment VIa: | Lys-Glu-Val-Val-Gla-Gla- |
| | 26 27 28 |
| | Ala-Glu-Asn |
| | 13 14 15 16 17 18 19 20 |
| Fragment VII: | Thr-Lys-Asp-Leu-Lys-Glu-Lys-Lys |
| | 21 22 23 24 25 26 27 28 |
| | Glu-Val-Val-Glu-Glu-Ala-Glu-Asn |
| | 7 8 9 10 11 12 13 14 |
| Fragment VIII: | Thr-Ser-Ser-Glu-Ile-Thr-Thr-Lys- |
| | 15 16 17 18 19 20 21 22 |
| | Asp-Leu-Lys-Glu-Lys-Lys-Glu-Val- |
| | 23 24 25 26 27 28 |
| | Val-Glu-Glu-Ala-Glu-Asn |
| | 1 2 3 4 5 6 7 |
| Fragment IX: | Ac-Ser-Asp-Ala-Ala-Val-Asp-Thr- |
| | 8 9 10 11 12 |
| | Ser-Ser-Glu-Ile-Thr |

Die oben angegebenen erfindungsgemässen Fragmente I bis IX können N-terminal eine Acylgruppe mit 1 bis 6 Kohlenstoffatomen, insbesondere eine Acetylgruppe oder eine Acylglycingruppe, deren Acylrest 1 bis 6 Kohlenstoffatome aufweisen kann, tragen. Dabei können bei den entsprechenden Thymosin$\alpha_1$-Fragmenten die Glutaminsäurereste 10, 21, 24, 25 und 27 sowie der Asparaginsäurerest 15 und der Asparaginrest 28 auch als Amid oder Alkylamid mit 1 bis 6 Kohlenstoffatomen in der Alkylgruppe bzw. als Diamid oder als Dialkylamid mit 1 bis 6 Kohlenstoffatomen in den Alkylgruppen vorliegen.

Dabei stehen die Acylgruppen mit 1 bis 6 Kohlenstoffatomen insbesondere für die Acetylgruppe, die Propionylgruppe und die Butyrylgruppe, während die bevorzugten Alkylgruppen mit 1 bis 6 Kohlenstoffatomen die Methylgruppe, die Äthylgruppe, die Propylgruppe und die verschiedenen Butylgruppen sind. Dabei können bei den entsprechenden Thymosin$\alpha_1$-Fragmenten die Position 1 anstelle von Serin durch einen Lysinrest und die Position 24 und 25 durch $\gamma$-Carboxyglutaminsäure (Gla) anstelle von Glutaminsäureresten variiert sein.

Charakteristische Merkmale der oben angegebenen erfindungsgemässen Fragmente sind in der nachstehenden Tabelle zusammengefasst:

Tabelle der $R_f$-Werte der Thymosin$\alpha_1$-Fragmente auf Kieselgel-Dünnschichtplatten (MERCK K 60 $F_{254}$, 0,25 mm).

| Fragment | Nr. | A+ | B+ | Spektren |
|---|---|---|---|---|
| Lys(2-6) | Ia | 9 | 28 | NMR |
| (7-12) | II | 24 | 38 | NMR |
| (13-19) | III | – | 8 | NMR |
| (20-24) | IV | – | 23 | NMR |
| (20-28) | VI | 5 | 12 | NMR |
| (13-18) | VII | 9 | 24 | NMR |
| (7-28) | VIII | 3 | – | – |
| Ac(1-12) | IX | – | 21 | – |
| (20-23)Gla | IVa | – | 31 | NMR |

+) $R_f \times 100$

Laufmittel A: Amylalkohol/Pyridin/Methyläthylketon/Ameisensäure/Wasser 40:28:11:5:15 (V/V)

Laufmittel B: n-Butanol/Pyridin/Eisessig/Wasser 5:5:1:4 (V/V)

Die in obiger Tabelle aufgeführten Spektren sind in den Fig. 3 bis 10 der Zeichnung dargestellt.

Gegenstand der Erfindung sind weiterhin die pharmazeutisch annehmbaren Salze der oben angegebenen Fragmente I bis IX.

Die erfindungsgemässen Thymosin$\alpha_1$-Fragmente können unter Anwendung üblicher Peptidsyntheseverfahren hergestellt werden, wie durch konventionelle Synthese in Lösung mit maximalem Schutz oder minimalem Schutz der Seitenketten, durch Festphasen-Synthese nach Merrifield, wobei das Peptid am polymeren Träger aufgebaut wird, oder nach der Polymerreagenzmethode, nach der das Peptid zur Kettenverlängerung über die polymergebundene aktivierte Aminosäure geleitet wird.

In besonders bevorzugter Weise bildet man jedoch die erfindungsgemässen Thymosin$\alpha_1$-Fragmente nach der Verfahrensweise der deutschen Patentanmeldung P 2 919 592.4 der Anmelderin. Nach dieser Verfahrensweise werden die erfindungsgemässen Thymosin$\alpha_1$-Fragmente durch schrittweise Sequenzverlängerung mit Hilfe der gemischten Anhydridmethode aufgebaut. Dabei werden insbesondere Schutzgruppen mit tertiärem Kohlenstoffatom in der Urethangruppierung, wie die tert.-Butyloxycarbonylgruppe (Abkürzung = Boc), die $\alpha,\alpha$-Dimethyl-3,5-dimethoxybenzyloxycarbonylgruppe (Ddz) und die 2-(p-Biphenyl)-propyl-2-oxy-carbonylgruppe (Bpoc) eingesetzt, die Nebenreaktionen durch sterische Hinderung unterdrücken und es ermöglichen, grössere Überschüsse des gemischten Anhydrids wiederholt anzuwenden.

In der beigefügten Zeichnung stellen dar:

Fig. 1 eine schematische Darstellung der Primärstruktur von Thymosin$\alpha_1$,

Fig. 2 eine schematische Darstellung der erfindungsgemässen Fragmente, wie sie im Zuge der Synthese von Thymosin$\alpha_1$ gemäss Patentanmeldung P 2 919 592.4 (co-pending US-Application of the same applicants claiming priority of the above German Application) zugänglich werden und

Fig. 3 bis 9 NMR-Spektren der Fragmente Ia, II, III, IV, VI, VII und IVa.

Die Erfindung sei im folgenden näher unter Bezugnahme auf die beigefügte Fig. 2 erläutert, die eine schematische Darstellung der erfindungsgemässen Thymosin$\alpha_1$-Fragmente mit den bei ihrer Herstellung angewandten Schutzgruppen wiedergibt. Die dort und in den Beispielen verwendeten Abkürzungen entsprechen den IUPAC-IUP-Vorschlägen (J. Biol. Chem. 247 (1972) 977-983). Dabei bedeuten:

Ddz: $\alpha,\alpha$-Dimethyl-3,5-dimethoxybenzyloxycarbonyl,
Z: Benzyloxycarbonyl,
Ac: Acetyl,
OBu$^t$: tert.-Butylester,
Mbh: 4,4'-Dimethyloxybenzhydryl,
Me: Methyl,
OBzl: Benzylester, und
Bu$^t$: tert.-Butyl.

Die folgenden Beispiele verdeutlichen die Herstellung der erfindungsgemässen Thymosin$\alpha_1$-Fragmente und ihre pharmakologische Wirksamkeit.

Die in den folgenden Beispielen angegebenen Herstellungsmethoden enden bei den geschützten Thymosin$\alpha_1$-Fragmenten, deren Schutzgruppen wie folgt abgespalten werden können:

Abspaltung der Schutzgruppen

Der Ddz-geschützte Peptidester wird mit dem 8- bis 10fachen Überschuss wasserfreier Trifluoressigsäure in Form einer 5%igen Lösung in Methylenchlorid (absolut) versetzt und während 15 bis 30 Minuten bei Raumtemperatur gerührt. Bei längeren Peptiden ist die Zeit zu verlängern. Ist die säurelabile 4,4'-Dimethoxybenzhydrylgruppe in der Sequenz enthalten, so wird die Ddz-Schutzgruppe mit 2,5%iger oder 1%iger Trifluoressigsäure während 30 bis 60 Minuten abgespalten. Zum Reaktionsabbruch wird die Lösung auf −15°C gekühlt und mit

der berechneten Menge N-Methylmorpholin (+ 10%) auf einen pH-Wert von 7,0 bis 7,5 gebracht.

Peptidmethylester werden in Alkohol, Dioxan, Tetrahydrofuran oder Dimethylformamid unter Zusatz von 20% Wasser in einem Ultraschallbad gelöst und dort mit 2 Äquivalent 1 n NaOH verseift. Nach dünnschichtchromatographisch ermitteltem Ende der Reaktionszeit wird mit Essigsäure neutralisiert, der Ansatz im Vakuum eingeengt und der Rückstand an Sephadex LH20 in Methanol chromatographiert.

Mit Trifluoressigsäure in Form einer 50%igen Lösung in Methylenchlorid unter Zusatz von Anisol werden primär die tert.-Butylester-Reste sowie die tert.-Butyläther-Gruppen abgespalten, während schliesslich dann die 4,4'-Dimethoxybenzhydryl-Schutzgruppe mit 95% Trifluoressigsäure/Anisol abgelöst wird. Enthalten die Fragmente Benzyloxycarbonyl-Schutzgruppen und/oder Benzylester, so werden diese vor der erwähnten Trifluoressigsäure-Behandlung in üblicher Weise durch Hydrieren in Gegenwart von Palladium auf Kohle in alkoholischer Lösung abgespalten.

Die erfindungsgemässen Thymosin$\alpha_1$-Fragmente können in üblicher Weise unter Anwendung von vorzugsweise pharmakologisch annehmbaren Säuren oder Basen in die entsprechenden Salze überführt werden.

*Beispiel 1*

*Darstellung von Ddz-(26-28)-Obzl*
*Ddz-(28)-OBzl, Ddz-Asn(Mbh)-OBzl*

11,6 g (20 mMol) Ddz-Asn(Mbh)-OH werden in 10 ml Dimethylformamid bei 0°C mit 3 ml Triäthylamin (10% Überschuss) und 3,48 g (= 2,43 ml) Benzylbromid versetzt. Die Lösung wird über Nacht bei Raumtemperatur gerührt und in 200 ml Eiswasser eingegossen. Die Suspension wird mit $NaHCO_3$-Lösung sofort neutralisiert, der Festkörper mit Äthylacetat in Lösung gebracht und die organische Phase mit $KHSO_4$-Lösung (kalt) und $NaHCO_3$-Lösung gewaschen. Wird in 93 : 7 noch freie Säure gefunden, so wird eine Kieselgelsäure mit $CHCl_3$/Äthanol 94 : 6 als Elutionsmittel zur Endreinigung angeschlossen. Ausbeute 8,2 g = 61%. Weitere Daten siehe Tabelle I.

*Ddz-(27-28)-OBzl, Ddz-Glu(OBu$^t$)-Asn(Mbh)-OBzl*

7 mMol Ddz-Glu(OBu$^t$)-OH werden gemäss der allgemeinen Arbeitsvorschrift zur gemischten Anhydridsynthese mit 10 mMol Asn(Mbh)-OBzl zum Dipeptid umgesetzt. Nach dem üblichen Ausschüttelvorgang wird über eine Kieselgelsäule mit $CHCl_3$/-Äthanol 93 : 7 chromatographiert. Das Dipeptid ist danach noch mit Ddz-Spaltstück verunreinigt, was aber weitere Syntheseoperationen nicht stört. Daten siehe Tabellen I und IX.

*Ddz-(26-28)-OBzl, Ddz-Ala-Glu(OBu$^t$)-Asn(Mbh)-OBzl*

15 mMol Ddz-Ala-OH werden mit 9,3 mMol Ddz-(27-28)-OBzl wie üblich zum Tripeptid umgesetzt. Es wird über Sephadex LH 20 mit Methanol und über Kieselgel mit $CHCl_3$/Äthanol 93 : 7 chromatographisch gereinigt. Ausbeute 50%. Weitere Daten siehe Tabellen I und IX.

*Beispiel 2*

*Thymosin$\alpha_1$-Fragment IV*
*Darstellung von Ddz-(20-24)-OMe*

a) Ddz-(24)-OMe, Ddz-Glu(OBu$^t$)-OMe

20 mMol Ddz-Glu(OBu$^t$)-OH werden in Aceton gelöst und mit der äquivalenten Menge Diazomethan (ätherische Lösung) versetzt. 100% Ausbeute eines gelblichen Öls, chromatographisch einheitlich. Weitere Daten siehe Tabelle II.

b) Ddz-(23-24)-OMe, Ddz-Val-Glu(OBu$^t$)-OMe

7,5 mMol Ddz-Val-OH werden mit 5 mMol Ddz-Glu(OBu$^t$)-OMe zum Dipeptid umgesetzt. Endreinigung über Kieselgel mit $CHCl_3$/Äthanol 93 : 7. Das Peptid enthält noch Ddz-Spaltstück, welches weitere Syntheseschritte nicht beeinflusst. Daten siehe Tabellen II und IX.

c) Ddz-(22-24)-OMe, Ddz-Val-Val-Glu(OBu$^t$)-OMe

Das Tripeptid wird auf üblichem Wege nach Chromatographie über Sephadex LH 20 mit Methanol rein erhalten. Es ist gut in Methanol und $CHCl_3$ löslich. 60% Ausbeute eines farblosen Öls. Weitere Daten siehe Tabelle II und IX.

d) Ddz-(21-24)-OMe, Ddz-Glu(OBu$^t$)-Val-Val-Glu(OBu$^t$)-OMe

6 mMol Ddz-Glu(OBu$^t$)-OH werden mit 2,98 mMol Ddz-(22-24)-OMe wie üblich zum Tetrapeptid umgesetzt. Es wird über eine Sephadex LH 20/Methanol-Säule gegeben, um in 94%iger Ausbeute das Produkt als farbloses Glas zu erhalten. Das Tetrapeptid löst sich gut in $CHCl_3$, Methanol, Dimethylformamid und Dioxan. Es ist dünnschichtchromatographisch in 93 : 7, 7 : 1 und 85 : 10 : 5 einheitlich und zeigt mit Ninhydrin die gewöhnliche violette Farbe. Weitere Daten siehe Tabellen II und IX.

e) Ddz-(20-24)-OMe, Ddz-Lys(Z)-Glu(OBu$^t$)-Val-Val-Glu(OBu$^t$)-OMe (geschütztes Fragment IV)

9 mMol Ddz-Lys(Z)-OH werden mit 2,43 mMol Ddz-(21-24)-OMe wie üblich zum Pentapeptid umgesetzt. Nach dem Ausschüttelvorgang wird über eine Methanol/Sephadex LH-20-Säule chromatographisch gereinigt, um in 99%iger Ausbeute das Produkt als farbloses Glas zu erhalten. Weitere Daten siehe Tabellen II und IX.

Durch übliche Abspaltung der Schutzgruppen erhält man das gewünschte Thymosin$\alpha_1$-Fragment IV.

*Beispiel 3*

*Thymosin$\alpha_1$-Fragment III*
*Darstellung von Ddz-(13-19)-OMe*

a) Ddz-(19)-OMe, Ddz-Lys(Z)-OMe

20 mMol Ddz-Lys(Z)-OH werden in Tetrahydrofuran gelöst und mit der äquivalenten Menge ätherischer Diazomethanlösung versetzt. Der Aminosäurerest wird in 95%iger Ausbeute als gelbliches Öl nach Ausschütteln mit $NaHCO_3$-Lösung erhalten. Eine Kristallisation aus den üblichen Lösungsmitteln gelang nicht. Weitere Daten siehe Tabelle III.

b) Ddz-(18-19)-OMe, Ddz-Glu(OBu$^t$)-Lys(Z)-OMe

Das Dipeptid wird auf üblichem Wege dargestellt. Man erhält das Peptid als gelbliches Öl, welches mit

geringen Mengen Ddz-Spaltstück verunreinigt ist. Daten siehe Tabellen III und X.

c) Ddz-(17-19)-OMe, Ddz-Lys(Z)-Glu(OBu$^t$)-Lys-(Z)-OMe

Die Synthese des Tripeptids verläuft nach der üblichen Methode normal. Es werden 20 mMol Ddz-Lys(Z)-OH mit 10 mMol Ddz-(18-19)-OMe umgesetzt. Nach der sauren und alkalischen Extraktion wird zuerst über Kieselgel mit $CHCl_3$/Äthanol 97 : 3, dann über Sephadex LH 20/Methanol chromatographiert. Ausbeute 86% eines farblosen, zerfliessenden Schaums. Weitere Daten siehe Tabellen III und X.

d) Ddz-(16-19)-OMe, Ddz-Leu-Lys(Z)-Glu(OBu$^t$)-Lys(Z)-OMe

Das Tetrapeptid wird auf üblichem Wege dargestellt. Nach Chromatographie über LH 20/Methanol können in 77%iger Ausbeute 4,1 g eines weissen, amorphen Festkörpers isoliert werden. Das Peptid ist in Methanol und $CHCl_3$ gut löslich. Weitere Daten siehe Tabellen III und X.

e) Ddz-(15-19)-OMe, Ddz-Asp(OBu$^t$)-Leu-Lys(Z)-Glu(OBu$^t$)-Lys(Z)-OMe

Zur Synthese des Pentapeptids ist die Abwandlung der allgemeinen Arbeitsvorschrift notwendig. Die gemischte Anhydridsynthese wird in der üblichen Stöchiometrie (9,5 mMol Ddz-Asp(OBu$^t$)-OH und 3,7 mMol Tetrapeptid) in einem Dreihalskolben mit kräftigem Rührwerk durchgeführt. Nach Zusammengeben der Carboxyl- und der Aminkomponente geliert die Methylenchloridlösung bei −15°C spontan. Eine Verflüssigung kann durch Zusatz von 40 bis 60 ml Dimethylformamid erreicht werden. Nach 4 bis 6 Stunden Reaktionszeit werden nochmals 100 bis 200 ml Dimethylformamid zugesetzt und das Methylchlorid vorsichtig abgedampft. Die zurückbleibende Dimethylformamidlösung wird langsam in das 5fache Volumen eiskalter NaCl-Lösung eingerührt und der weisse, flockige Niederschlag abgesaugt. Nach Waschen mit Wasser, Wiederholung der Fällung und Trocknung erhält man in 84%iger Ausbeute das Peptid. Reste des Ddz-Spaltstücks können mit Äther ausgewaschen werden.

Das Peptid ist sehr wenig löslich in Methanol, $CH_2Cl_2$, $CHCl_3$ und Dioxan. In 1,1-Dichloräthan kann eine Suspension hergestellt werden. In 5%iger Trifluoressigsäure/$CH_2Cl_2$ ist das Peptid ohne Probleme löslich. Weitere Daten siehe Tabellen III und X.

f) Ddz-(14-19)-OMe, Ddz-Lys(Z)-Asp(OBu$^t$)-Leu-Lys(Z)-Glu(OBu$^t$)-Lys(Z)-OMe

3,5 mMol Ddz-Lys(Z)-OH und 1,74 mMol Ddz-(15-19)-OMe können ohne weitere Löslichkeitsprobleme zum Hexapeptid umgesetzt werden. Nach der Chromatographie über Sephadex LH 20/Methanol wird in ca. 100%iger Ausbeute ein glasartiger Festkörper erhalten. Das Produkt ist gut in Methanol, besonders nach Dimethylformamid-Zusatz, löslich. Weitere Daten siehe Tabellen III und X.

g) Ddz-(13-19)-OMe, Ddz-Thr(Bu$^t$)-Lys(Z)-Asp-(OBu$^t$)-Leu-Lys(Z)-Glu(OBu$^t$)-Lys(Z)-OMe (geschütztes Fragment III)

11,4 mMol Ddz-Thr(Bu$^t$)-OH werden mit 5,7 mMol Ddz-(14-19)-OMe umgesetzt. Dabei ist zu beachten, dass das Heptapeptid zur sauer/alkalischen Extraktion in $CH_2Cl_2$ gelöst bleibt, also der übliche Lösungsmittelwechsel entfällt. Das Peptid ist nur wenig in Äthylacetat, Methanol und Dimethylformamid löslich, dagegen gut in $CH_2Cl_2$ und $CHCl_3$. Chromatographische Reinigung erfolgt über Kieselgel und $CHCl_3$/Äthanol 98 : 2 als Elutionsmittel, wonach das Heptapeptid in 56%iger Ausbeute erhalten wird. Es färbt mit Ninhydrin auffällig gelbrot an. Weitere Daten siehe Tabellen III und X.

h) Durch Abspaltung der Schutzgruppen erhält man das gewünschte Thymosin$\alpha_1$-Fragment III.

*Beispiel 4*

*Thymosin$\alpha_1$-Fragment II*
*Darstellung von Ddz-(7-12)-OBzl*

a) Ddz-(12)-OBzl, Ddz-Thr(Bu$^t$)-OBzl

20 mMol Ddz-Thr(Bu$^t$)-OH werden analog der Vorschrift für Ddz-Asn(Mbh)-OBzl mit Benzylbromid verestert. Durch Extraktion mit $KHSO_4$- und $KHCO_3$-Lösung kann ein chromatographisch einheitliches Öl in 73%iger Ausbeute erhalten werden. Weitere Daten siehe Tabelle IV.

b) Ddz-(11-12)-OBzl, Ddz-Ile-Thr(Bu$^t$)-OBzl

Nach Ddz-Abspaltung aus 10 mMol Ddz-Thr(Bu$^t$)-OBzl wird mit 15 mMol Ddz-Ile-OH auf üblichem Wege das Dipeptid dargestellt. Es wird über Kieselgel mit Chloroform/Äthanol 98 : 2 chromatographiert und aus $CCl_4$/n-Heptan kristallisiert. 5,1 g feine weisse Nadeln (= 8,47 mMol, 85%) wurden so gewonnen. Weitere Daten siehe Tabelle IV.

c) Ddz-(10-12)-OBzl, Ddz-Glu(OBu$^t$)-Ile-Thr(Bu$^t$)-OBzl

16 mMol Ddz-Glu(OBu$^t$)-OH werden mit 8 mMol Ddz-(11-12)-OBzl auf übliche Weise zum Tripeptid umgesetzt. Nach saurer oder alkalischer Extraktion wird mit Methanol über LH 20 chromatographiert. Ausbeute 89%. Weitere Daten siehe Tabelle IV.

d) Ddz-(9-12)-OBzl, Ddz-Ser(Bu$^t$)-Glu(OBu$^t$)-Ile-Thr(Bu$^t$)-OBzl

Wie üblich wird aus 7 mMol Ddz-(10-12)-OBzl mit 14 mMol Ddz-Ser(Bu$^t$)-OH das Tetrapeptid hergestellt. Nach saurer und alkalischer Extraktion wird die Reinigung über eine Kieselgelsäule ($CHCl_3$/Äthanol 98 : 2) vorgenommen. Man erhält 5,3 g einer weissen amorphen Substanz (5,8 mMol, 83%) mit einem Schmelzbereich von 172 bis 177°C. Weitere Daten siehe Tabelle IV.

e) Ddz-(8-12)-OBzl, Ddz-Ser(Bu$^t$)-Ser(Bu$^t$)-Glu-(OBu$^t$)-Ile-Thr(Bu$^t$)-OBzl

Aus 11,6 mMol Ddz-Ser(Bu$^t$)-OH und 5,8 mMol Ddz-(19-12)-OBzl wird wie üblich das Pentapeptid erhalten. Nach saurer und alkalischer Extraktion wird in Methanol/Dimethylformamid 8 : 2 gelöst und auf eine Sephadex LH 20/Methanol-Säule aufgetragen. Im Eluat kristallisiert spontan das in Methanol schwerlösliche Peptid aus. 4,7 g weisse Nadeln werden erhalten, das sind 78% Ausbeute. Weitere Daten siehe Tabellen IV und X.

f) Ddz-(7-12)-OBzl, Ddz-Thr(Bu$^t$)-Ser(Bu$^t$)-Ser-(Bu$^t$)-Glu(OBu$^t$)-Ile-Thr(Bu$^t$)-OBzl (geschütztes Fragment II)

Aus 6,2 mMol Ddz-Thr(Bu$^t$)-OH und 3,5 mMol Ddz-(8-12)-OBzl wird auf üblichem Wege das Hexapeptid erhalten. Nach saurer und alkalischer Extraktion wird das in Methanol schwerlösliche Peptid in

Dimethylformamid/Methanol gelöst und auf eine LH 20-Säule aufgetragen und mit Methanol eluiert. Man erhält 4,0 g einer glasig amorphen Substanz, die mit Ninhydrin gelbrot anfärbt, Ausbeute = 97%. Das Peptid ist löslich in Benzol, $CH_2Cl_2$, $CHCl_3$ und Dimethylformamid. Weitere Daten siehe Tabellen IV und X.

g) In analoger Weise erhält man

Ddz-(7-12)-OMe, Ddz-Thr($Bu^t$)-Ser($Bu^t$)-Ser($Bu^t$)-Glu($OBu^t$)-Ile-Thr($Bu^t$)-OMe (geschütztes Fragment II)

Die Synthese verläuft vollkommen analog zu der Präparation des Ddz-(7-12)-Benzylesters. Alle Eigenschaften der Zwischenprodukte können aus Tabelle V bzw. Tabelle I entnommen werden.

h) Durch Abspalten der Schutzgruppen erhält man das gewünschte Thymosin$\alpha_1$-Fragment II.

*Beipsiel 5*

*Thymosin$\alpha_1$-Fragment I*
*Darstellung von Ac-(1-6)-OBzl*

a) Ddz-(6)-OBzl, Ddz-Asp($OBu^t$)-OBzl

20 mMol Ddz-Asp($OBu^t$)-OH werden analog der Vorschrift für Ddz-(28)-OBzl mit Benzylbromid verestert. Durch Extraktion mit $KHSO_4$-Lösung und $KHCO_3$-Lösung kann ein chromatographisch einheitliches Öl in 81%iger Ausbeute erhalten werden. Daten siehe Tabelle VI.

b) Ac-(1)-OH, Ac-Ser($Bu^t$)-OH

Aus 20 mMol Ddz-Ser($Bu^t$)-OH wird mit 5%iger Trifluoressigsäure Ddz abgespalten und das Lösungsmittel im Vakuum abgezogen. Bei 0°C wird mit 20 ml 1n NaOH gelöst, in einen Dreihalskolben überführt. Am pH-Staten werden bei pH 9 200 mMol (= 15,6 g) Acetylchlorid 1 : 1 mit Dioxan verdünnt zugetropft, wobei die Temperatur bei 0 bis 4°C gehalten wird. 30 Minuten nach beendeter Zugabe werden im Vakuum ca. 50 ml eines Dioxan/$H_2O$-Azeotrops abgezogen. Die alkalische Lösung wird mit Äther extrahiert, mit $KHSO_4$ auf pH 2 bis 3 gebracht und nochmals mit Äther gewaschen. Schliesslich wird das Produkt mit Äthylacetat aus der wässrigen Phase extrahiert. Es werden 3,6 g = 96% einer amorphen Substanz erhalten, die aus Äthylacetat im Kühlschrank farblos körnig kristallisiert. Weitere Daten siehe Tabelle VI.

c) Ddz-(5-6)-OBzl, Ddz-Val-Asp($OBu^t$)-OBzl

15 mMol Ddz-Val-OH werden mit 11,5 mMol Ddz-Asp($OBu^t$)-OBzl auf üblichem Wege umgesetzt. Das Dipeptid wird über Kieselgel mit $CHCl_3$/Äthanol 99 : 1 chromatographiert. Daten siehe Tabellen VI und X.

d) Ddz-(4-6)-OBzl, Ddz-Ala-Val-Asp($OBu^t$)-OBzl

20 mMol Ddz-Ala-OH werden ohne Probleme mit Ddz-(5-6)-OBzl Tripeptid umgesetzt. Chromatographische Reinigung über Sephadex LH 20/Methanol. Daten siehe Tabellen VI und X.

e) Ddz-(3-6)-OBzl, Ddz-Ala-Ala-Val-Asp($OBu^t$)-OBzl

Aus 17 mMol Ddz-Ala-OH und 8,8 mMol Ddz-(4-6)-OBzl wird auf übliche Weise das Tetrapeptid hergestellt. Bei der sauren und alkalischen Extraktion flockt das Peptid aus Äthylacetat aus, kann aber in Suspension gehalten werden. Eine Gelchromatographie (LH 20) liefert kein chromatographisch einheitliches Produkt. Daher wird noch über Kieselgel in $CHCl_3$/Äthanol 99 : 1 chromatographiert. Man erhält in 70%iger Ausbeute 4,5 g eines farblosen amorphen Produkts. Weitere Daten siehe Tabellen VI und X.

f) Ddz-(2-6)-OBzl, Ddz-Asp($OBu^t$)-Ala-Ala-Val-Asp($OBu^t$)-OBzl

Bei der üblichen Umsetzung von 11,6 mMol Ddz-Asp($OBu^t$)-OH mit 5,8 mMol Ddz-(3-6)-OBzl fällt die Schwerlöslichkeit der Aminkomponente in $CH_2Cl_2$ auf. Gelartig ausgefallenes Peptid kann nach der Ddz-Abspaltung und während der Kupplung mit Dimethylformamid in Lösung gebracht werden. Das rohe Pentapeptid wird in Methanol/Dimethylformamid gelöst und über LH 20 chromatographiert. Dabei kristallisiert es aus den Fraktionen der Chromatographie in feinen weissen Nadeln aus. Ausbeute 85%. Daten siehe Tabellen VI und X.

g) Ac-(1-6)-OBzl, Ac-Ser($Bu^t$)-Asp($OBu^t$)-Ala-Ala-Val-Asp($OBu^t$)-OBzl (geschütztes Fragment I)

2,8 mMol Ac-Ser($Bu^t$)-OH werden wie üblich mit 1,4 mMol Ddz-(2-6)-OBzl umgesetzt. Nach beendeter Reaktion und Abziehen des $CH_2Cl_2$ wird in 150 ml Dimethylformamid gelöst und in 800 ml eiskalte, mit NaCl gesättigte 5%ige $NaHCO_3$-Lösung eingerührt. Das ausgefallene Produkt wird abgesaugt, mit Wasser gewaschen und getrocknet. Es wird in wenig $CHCl_3$ gelöst und auf eine MERCK-Fertigsäule aufgetragen. Diese wird mit alkoholfreiem $CHCl_3$ eluiert und nach Durchtreten der Front mit einem linearen Gradienten aus $CHCl_3$/O bis 10% Äthanol entwickelt. Man erhält 950 mg weiss-amorphes Peptid. Es ist nicht gut in $CHCl_3$ löslich, lässt sich nicht mit Ninhydrin anfärben, reagiert aber mit «Chlor I/II». Daten siehe Tabellen VI und X.

h) Herstellung von Ddz-(1-6)-OMe

Das Hexapeptid wird analog den Arbeitsvorschriften für Ac-(1-6)-OBzl dargestellt.

Ddz-Ser($Bu^t$)-Asp($OBu^t$)-Ala-Ala-Val-Asp($OBu^t$)-OMe

Das Hexapeptid löst sich gut in Methanol/Dimethylformamid. Es kann problemlos über Sephadex LH 20 chromatographiert werden. Seine Löslichkeit in $CHCl_3$, Tetrahydrofuran, Dioxan ist mässig gut, jedoch deutlich besser als das Acetylpeptid.

Gleiche Eigenschaften besitzt auch das im folgenden beschriebene Fragment Ddz-(1-6)-OBzl.

i) Darstellung von Ddz-(1-6)-OBzl

Das Hexapeptid wird analog der Arbeitsvorschrift für Ac-(1-6)-OBzl hergestellt unter Verwendung von Ddz-Ser($Bu^t$) anstelle von Ac-Ser($Bu^t$).

j) Darstellung von Ddz-Lys(Z)-(2-6)-OBzl; (geschütztes Fragment Ia)

7 mMol Ddz-Lys(Z)-OH und 3,1 mMol Ddz-(2-6)-OBzl werden wie üblich zum Hexapeptid umgesetzt. Nach Chromatographie über Sephadex LH 20/Methanol wird nahezu quantitativ das einheitliche Endprodukt erhalten. Daten siehe Tabellen VI und XI.

k) Durch Abspalten der Schutzgruppen erhält man aus den unter i) und j) genannten Produkten das gewünschte Thymosin$\alpha_1$-Fragment I bzw. Ia, Lys-(2-6)-OH.

*Beispiel 6*

*Thymosin$\alpha_1$-Fragment VII*
*Synthese des Fragments Ddz-(13-28)-OBzl*

a) Verseifung von Ddz-(20-24)-OMe, Ddz-Lys(Z)-Glu(OBu$^t$)-Val-Val-Glu(OBu$^t$)-OMe

1 mMol (1,1 g) Ddz-(20-24)-OMe werden in 20 ml wässrigem 80%igem Dioxan (Konzentration 50 mMol) gelöst. Ein Ultraschallbad ist hierbei von Vorteil. Dann werden 2 ml (2 mMol) 1 n NaOH zugesetzt und eine Stunde gerührt. Die Reaktionszeit wird durch einen Vorversuch erprobt. Nach Abbruch der Verseifung mit 1 mMol Essigsäure wird ein Teil des Dioxans am Rotationsverdampfer abgezogen und der Rückstand über eine Sephadex LH 20/Methanol-Säule in einen Peptid- und einen Salzpeak aufgetrennt. Der Peptidpeak enthält in 76%iger Ausbeute die chromatographisch reine Peptidsäure. Weitere Daten siehe Tabellen VII und XI.

b) Fragmentkondensation zu Ddz-(20-28)-OBzl (geschütztes Fragment VI)

Aus 895 mg (= 0,86 mMol, 15% Überschuss) Ddz-(25-28)-OBzl wird in 45minütiger Reaktion mit einem 8fachen Überschuss 2,5%iger Trifluoressigsäure in $CH_2Cl_2$ die Schutzgruppe abgespalten. Reaktionsabbruch und Neutralisation (pH 7,5) erfolgt mit 1,0 ml N-Methylmorpholin. Das Lösungsmittel wird im Vakuum eingedampft.

800 mg (0,76 mMol) Ddz-(20-24)-OH werden in 15,2 ml Dimethylformamid (Endkonzentration 50 mM) gelöst, mit der Aminkomponente versetzt und auf 0°C gekühlt. Dann werden 1,52 mMol (= 313 mg, 2 Äquivalente) Dicyclohexylcarbodiimid und 349 mg (2,28 mMol, 3 Äquivalente) 1-Hydroxybenzotriazol·$H_2O$ zugesetzt. Die klare Lösung wird eine Stunde bei 0°C gerührt, dann langsam auf Raumtemperatur erwärmt. Nach einer weiteren Stunde fällt plötzlich Dicyclohexylharnstoff in mikrokristallinen Schlieren aus. Nach insgesamt 24 Stunden Reaktionszeit wird auf 0°C gekühlt, vom Dicyclohexylharnstoff abzentrifugiert und in 50 ml 5%iger $NaHCO_3$-Lösung gefällt, der Niederschlag mit Eiswasser gewaschen und getrocknet.

Die Rohsubstanz (0,7 mMol = 93%) wird in $CHCl_3$ gelöst und auf einer MERCK-Fertigsäule K 60 Grösse C, die mit $CHCl_3$ äquilibriert ist, aufgetragen. Es wird ein flacher Gradient von $CHCl_3$ gegen $CHCl_3$/Äthanol 90 : 10 angelegt. Es werden 1,1 g (= 70%) reinen Nonapeptids erhalten. Weitere Daten siehe Tabellen VIII und XI.

c) Verseifung von Ddz-(13-19)-OMe, Ddz-Thr(Bu$^t$)-Lys(Z)-Asp(OBu$^t$)-Leu-Lys(Z)-Glu(OBu$^t$)-Lys(Z)-OMe

0,7 mMol Ddz-(13-19)-OMe (= 1,2 g) werden mit 9 ml Tetrahydrofuran und 4 ml Methanol unter Einwirkung von Ultraschall gelöst, dann mit 2 ml Wasser versetzt (Endkonzentration 47 mM). Nach Zusatz von 1,5 ml 1 n NaOH (1,4 mMol) wird 25 Minuten verseift. Die Reaktionszeit wird durch einen Vorversuch ermittelt. Der Reaktionsabbruch erfolgt mit 0,7 mMol Essigsäure. Nach Konzentrierung wird über Sephadex LH 20/Methanol chromatographiert. Weitere Daten siehe Tabellen VII und XI.

d) Fragmentkondensation zu Ddz-(13-28)-OBzl (geschütztes Fragment VII)

Aus 583 mg (= 0,3 mMol) Ddz-(20-28)-OBzl wird in 45minütiger Reaktion mit einem 10fachen Überschuss 2,5%ige Trifluoressigsäure in $CH_2Cl_2$ die Ddz-Schutzgruppe abgespalten. Reaktionsabbruch und Neutralisation erfolgen mit 0,33 ml N-Methylmorpholin (pH 7,5). Das Lösungsmittel wird im Vakuum abgezogen.

663 mg (= 0,4 mMol, 1,33 Äquivalente) Ddz-(13-19)-OH werden in 12 ml Dimethylformamid (Endkonzentration 25 mM) gelöst, mit der Aminkomponente versetzt und auf 0°C gekühlt. Dann werden 165 mg (0,8 mMol) Dicyclohexylcarbodiimid und 184 mg (= 1,2 mMol) 1-Hydroxybenzotriazol·$H_2O$ zugesetzt. Die klare und nochmals mit N-Methylmorpholin auf pH 7,5 eingestellte Lösung wird eine Stunde bei 0°C gerührt, dann langsam auf Raumtemperatur erwärmt. Nach weiteren 1,5 bis 2 Stunden fällt plötzlich Dicyclohexylharnstoff in mikrokristallinen Schlieren aus. Nach insgesamt 66 Stunden (bei einem zweiten Ansatz 24 Stunden) wird auf 0°C gekühlt, vom Dicyclohexylharnstoff abzentrifugiert und über eine grosse LH 20/Methanol-Säule chromatographiert. Aus den ersten peptidhaltigen Fraktionen fällt ein weisses flockiges Material aus, welches als Hexadekapeptid identifiziert wird. Der zweite und dritte Peak der Trennung enthält nichtumgesetzte Carboxyl- bzw. Aminkomponente.

Das Hexadekapeptid wird als weisses Pulver erhalten, welches in Methanol wenig, nach Dimethylformamid-Zusatz jedoch gut löslich ist. Es zeigt bei 230°C beginnende Zersetzung, die bei 260°C vollständig ist. Weitere Daten siehe Tabellen VIII und XI.

e) Durch übliches Abspalten der Schutzgruppen erhält man das gewünschte Thymosin$\alpha_1$-Fragment VII.

*Beispiel 7*

*Thymosin$\alpha_1$-Fragment IX*

a) Hydrierung von Ac-(1-6)-OBzl, Ac-Ser(Bu$^t$)-Asp(OBu$^t$)-Ala-Ala-Val-Asp(OBu$^t$)-OBzl

500 mg Ac-(1-6)-OBzl werden in 100 ml Isopropanol/10% Essigsäure gequollen und mit 600 ml Isopropanol bei 50°C im Vibromischer gelöst. Die Hydrierung wird bei Raumtemperatur am Vibromischer mit 1 g Pd/C im $H_2$-Strom durchgeführt. Nach 9 Stunden wird vom Katalysator abgenutscht und das Lösungsmittel unter Zusatz von Toluol im Vakuum abgezogen. Das Rohprodukt wird in Methanol unter Zusatz von etwas Dimethylformamid auf eine LH 20-Säule aufgetragen und chromatographiert. Ausbeute 300 mg = 67%.

Die Hydrierung kann auch in 50 ml 2,2,2-Trifluoräthanol mit 0,5 g Pd/C, die Reinigung auch über LH 20/2,2,2-Trifluoräthanol durchgeführt werden. Analysedaten siehe Tabellen VII und XI.

b) Fragmentkondensation zu Ac-(1-12)-OBzl (geschütztes Fragment IX)

1,54 g (= 1,3 mMol) Ddz-(7-12)-OBzl werden in 15 ml $CH_2Cl_2$ gelöst und zur Ddz-Schutzgruppenabspaltung mit 0,77 ml Trifluoressigsäure (8facher Überschuss, 5%ige Lösung) versetzt. Nach 15 Minuten wird mit 1,4 ml N-Methylmorpholin (1,2 Äquivalente) neutralisiert (pH 7,5).

900 mg Ac-(1-6)-OH (= 1,17 mMol) werden zusammen mit der Aminkomponente in 35 ml Dimethylformamid und 11 ml N-Methylmorpholin gelöst und auf 0°C gekühlt. Es werden 2,34 mMol (= 2 Äquivalente) Dicyclohexylcarbodiimid und 3,51 mMol (= 3 Äquivalente) 1-Hydroxybenzotriazol·$H_2O$ zugesetzt und eine Stunde bei 0°C gerührt. Während der 60 Stunden Reaktionszeit bei Raumtemperatur beobachtet man eine zunehmende Trübung der Lösung durch gelartig ausfallendes Dodekapeptid. Es wird zentrifugiert, der Niederschlag mit Dimethylformamid und Wasser je zweimal gewaschen und getrocknet. Es wurden 1,4 g eines weissen Pulvers erhalten. Dies entspricht 0,72 mMol, d.h. einer Ausbeute von 61% der Theorie.

Das Produkt ist weitgehend unlöslich: $CH_2Cl_2$, $CHCl_3$, Dioxan, Tetrahydrofuran, Methanol, Dimethylformamid, N-Methylpyrrolidon, Hexamethylphosphorsäure-triamid, tert.-Butanol, Isoamylalkohol, Isopropanol, Cyclohexan, Cyclohexanon, Zusatz von Ac-OH, Äther, Benzin und andere lösen das Peptid nicht. Nur in 2,2,2-Trifluoräthanol ist es löslich. Weitere Daten siehe Tabellen VIII und XI.

c) Hydrierung von Ac-(1-12)-OBzl

566 mg Ac-(1-12)-OBzl werden mit 100 mg Pd/C in 60 ml destilliertem Trifluoräthanol über Nacht hydriert.

Gereinigt wird über eine Kieselgel-Fertigsäule MERCK mit einem Gradienten von $CHCl_3$/2,2,2-Trifluoräthanol/Essigsäure 85 : 10 : 5 nach 2,2,2-Trifluoräthanol/Essigsäure 90 : 10. Die Substanz eluiert in einem flachen Peak gegen Ende des Gradienten. Daten siehe Tabellen VII und XI.

d) Durch Abspalten der Schutzgruppen erhält man das gewünschte Thymosin$\alpha_1$-Fragment IX.

*Beispiel 8*

*Thymosin$\alpha_1$-Fragment VIII*

a) Verseifung des Ddz-(7-12)-OMe, Ddz-Thr-(Bu$^t$)-Ser(Bu$^t$)-Ser(Bu$^t$)-Glu(OBu$^t$)-Ile-Thr(Bu$^t$)-OMe

1,0 g (0,95 mMol) Ddz-(7-12)-OMe werden in 19 ml Dioxan gelöst und mit 2,5 ml $H_2O$ versetzt. Fällt dabei die Substanz aus, so kann durch Ultraschallbehandlung eine für die Verseifung günstige Emulsion erhalten werden. Die Reaktion wird mit 0,5 ml 1 n NaOH gestartet. Nach 20 und 40 Minuten werden weitere 0,5 ml bzw. 0,9 ml 1 n NaOH zugesetzt. Die Reaktion wird nach 1 Stunde mit 0,95 mMol Essigsäure abgestoppt (pH 7,5).

Das Lösungsmittel wird im Vakuum abgezogen, in Methanol gelöst und über LH 20/Methanol chromatographiert. Dabei eluiert nichtumgesetzter Peptidester vor dem Peptidsäure-Hauptpeak. Die Substanz (57% Ausbeute) ist gut in Methanol, $CHCl_3$ und Toluol/Methanol löslich. Weitere Daten siehe Tabellen VII und XI.

b) Fragmentkondensation zu Ddz-(7-28)-OBzl (geschütztes Fragment VIII)

In einem typischen Ansatz werden aus 120 mg Ddz-(13-28)-OBzl (36 $\mu$Mol) in einstündiger Reaktion mit einem 20fachen Überschuss 2,5%iger (oder 1%iger) Trifluoressigsäure in $CH_2Cl_2$ die Ddz-Schutzgruppe abgespalten. Reaktionsabbruch und Neutralisation (pH 7,5) erfolgen mit N-Methylmorpholin (Mikroliterspritze). Das Lösungsmittel wird im Vakuum abgezogen.

77 mg (72 $\mu$Mol, 2 Äquivalente) Ddz-(7-12)-OH werden in 1,5 ml Dimethylformamid gelöst, mit der Aminkomponente versetzt und auf 0°C gekühlt. Dann werden 16 mg (78,6 $\mu$Mol, 2,2 Äquivalente) Dicyclohexylcarbodiimid und 24 mg (157 $\mu$Mol, 4,4 Äquivalente) 1-Hydroxybenzotriazol·$H_2O$ zugesetzt und der pH mit N-Methylmorpholin (theoretisch 6,4 Äquivalente) auf 7,5 eingestellt. Die klare Lösung wird über Nacht bei 0°C gerührt. Ausfallen des Dicyclohexylharnstoffs zeigt das Fortschreiten der Reaktion an. Es wird noch 10 Stunden bei ca. 20°C gehalten, wieder auf 0°C gekühlt und der Dicyclohexylharnstoff abzentrifugiert. Der Überstand wird in 30 ml eiskaltes Methanol getropft, worauf das rohe 22-Peptid ausfällt. Es wird im Vakuum getrocknet, in 2,2,2-Trifluoräthanol gelöst und über eine 2,2,2-Trifluoräthanol/LH 20-Säule (0,6 × 240 cm) chromatographisch gereinigt, indem der erste Peak bis zur Einheitlichkeit rechromatographiert wird.

Durch übliches Abspalten der Schutzgruppen erhält man das gewünschte Thymosin$\alpha_1$-Fragment VIII.

Das Peptid wird als glasartiger Festkörper erhalten, welcher in Methanol unlöslich, in Dimethylformamid löslich und in 2,2,2-Trifluoräthanol leicht löslich ist. Die Ausbeute beträgt 67%. Weitere Daten siehe Tabellen VIII und XI.

TABELLE I

| | Ausbeute | Aussehen | Fp (°C) | hRF I | hRF II | $[\alpha]^{20}$ 578 nm | $[\alpha]^{20}$ 265 nm | (g/100 ml) | Lösungsmittel |
|---|---|---|---|---|---|---|---|---|---|
| Ddz-(28)-OBzl | 61% | weiss amorph | 136-138 | 49 | 92 | −4,4° | −12,3° | 1,145 | Methanol |
| Ddz-(27-28)-OBzl | 100% | gelbl. krist. | 125-132 | 35 | 92 | −4,8° | −15,6° | 1,465 | Methanol |
| Ddz-(26-28)-OBzl | 50% | weiss schaumig | | | | −23,8° | −80,3° | 1,333 | Methanol |

I: 7 : 1, II: 85 : 10 : 5

TABELLE II

Thymosin$\alpha_1$-Fragment IV

| | Ausbeute | Aussehen | hRf I | hRf II | $[\alpha]^{20}$ 578 nm | 365 nm | c(g/100 ml) | Lösungsmittel |
|---|---|---|---|---|---|---|---|---|
| Ddz-(24)-OMe | 100% | farbl. Öl | 57 | 92 | | | | |
| Ddz-(23-24)-OMe | 100% | Öl | 47 | 92 | | | | |
| Ddz-(22-24)-OMe | 60% | farbl. Öl | 28 | 92 | | | | |
| Ddz-(21-24)-OMe | 94% | farbl. schaumig | 22 | 88 | | | | |
| Ddz-(20-24)-OMe | 99% | farbl. Glas | 05 | 92 | −44,5° | −155,9° | 0,53 | Methanol |

I: 7 : 1, II: 85 : 10 : 5

TABELLE III

Thymosin$\alpha_1$-Fragment III

| | Ausbeute | Aussehen | Fp (°C) | hRf I | hRf II | hRf III | hRf IV | $[\alpha]^{20}$ 578 nm | 365 nm | c(g/100 ml) | Lösungsmittel |
|---|---|---|---|---|---|---|---|---|---|---|---|
| Ddz-(19)-OMe | 95% | gelbl. Öl | — | 45 | | 92 | | −6,2° | −16,4° | 1,000 | Methanol |
| Ddz-(18-19)-OMe | 100% | gelbl. Öl | — | 31 | | 90 | | −12,8° | | 1,000 | Methanol |
| Ddz-(17-19)-OMe | 86% | zerfl. Schaum | — | 15 | | 86 | 15 | −18,5° | −61,3° | 1,539 | Methanol |
| Ddz-(16-19)-OMe | 77% | weiss schaumig | — | 21 | | 84 | | −29,7° | −100,0° | 0,740 | Methanol |
| Ddz-(15-19)-OMe | 84% | weiss amorph | 184-185 (G.) | 16 | | 85 | | −21,8° | −70,0° | 0,370 | Dimethylformamid |
| Ddz-(14-19)-OMe | 100% | glasartig farbl. | — | 01 | | 92 | | −23,4° | −81,9° | 0,475 | Methanol |
| Ddz-(13-19)-OMe | 56% | glasartig farbl. | — | 00 | 55 | 88 | | −24,5° | −87,2° | 0,375 | Methanol |

I: 7 : 1, II: 93 : 7, III: 85 : 10 : 5, IV: 4 : 1 : 1

TABELLE IV

Thymosin$\alpha_1$-Fragment II

| | Ausbeute | Aussehen | Fp (°C) | hRf I | hRf II | hRf III | $[\alpha]^{20}$ 578 nm | 365 nm | c(g/100 ml) | Lösungsmittel |
|---|---|---|---|---|---|---|---|---|---|---|
| Ddz-(12)-OBzl | 73% | farbl. Öl | — | 58 | | 59 | −17,5° | −53,5° | 2,017 | Methanol |
| Ddz-(11-12)-OBzl | 85% | feine Nadeln | 116,5-117,5° | 57 | 83 | | −20,6° | −69,1° | 0,33 | Methanol |
| Ddz-(10-12)-OBzl | 89% | farbl. Schaum | — | 38 | 92 | | | | | |
| Ddz-(9-12)-OBzl | 83% | weiss amorph | 172-177° | 34 | 93 | | −4,3° | −19,8° | 0,515 | $CH_2Cl_2$ |
| Ddz-(8-12)-OBzl | 78% | weisse Nadeln | 191-192° | 28 | 95 | | −2,2° | −16,5° | 0,87 | $CH_2Cl_2$ |
| Ddz-(7-12)-OBzl | 97% | glasig amorph | | 17 | 92 | 58 | −5,1° | −6,9° | 0,505 | $CH_2Cl_2$ |

I: 7 : 1, II: 85 : 10 : 5, III: 93 : 7

TABELLE V

Thymosin$\alpha_1$-Fragment II

| | Ausbeute | hRf I | hRf II | $[\alpha]^{20}$ 578 nm | 365 nm | c(g/100 ml) | Lösungsmittel |
|---|---|---|---|---|---|---|---|
| Ddz-(11-12)-OMe | 68,0% | 53 | 92 | −22,6° | −73,1° | 2 | Methanol |
| Ddz-(10-12)-OMe | 77,9% | 37 | 81 | −15,9° | −52,3° | 2 | Methanol |
| Ddz-(9-12)-OMe | 62,3% | 22 | 73 | −5,9° | −24,1° | 1 | Methanol |
| Ddz-(8-12)-OMe | 84,2% | 11 | 72 | −1,0° | −11,8° | 1 | Methanol |
| Ddz-(7-12)-OMe | 71,4% | 12 | 78 | −1,6° | −11,0° | 1 | Methanol |

I: 7 : 1, II: 93 : 7

TABELLE VI

Thymosin$\alpha_1$-Fragment I

| | Ausbeute | Aussehen | hRf I | II | III | IV | V | $[\alpha]^{20}$ 578 nm | 365 nm | c(g/100 ml) | Lösungsmittel |
|---|---|---|---|---|---|---|---|---|---|---|---|
| Ddz-(6)-OBzl | 81% | farbl. Öl | 48 | | 40 | | | −19,8° | −70,7° | 1,388 | Methanol |
| Ac-(1)-OH | 96% | körnige Krist. | 07 | | | 71 | 38 | +39,2° | +133,3° | 0,354 | Äthylacetat |
| Ddz-(5-6)-OBzl | 100% | gelbl. Öl | 58 | 92 | | | | −6,5° | −23,7° | 8,44 | Methanol |
| Ddz-(4-6)-OBzl | 76% | Schaum | 27 | 92 | | | | −47,4° | −171,2° | 0,515 | Methanol |
| Ddz-(3-6)-OBzl | 70% | farbl. amorph | 24 | 78 | | | | −54,6° | −193,4° | 0,71 | Methanol |
| Ddz-(2-6)-OBzl | 85% | Nadeln | 05 | 92 | | | | −24,6° | −97,7° | 0,435 | $CH_2Cl_2$ |
| Ac-(1-6)-OBzl | 78% | weiss schaumig | 00 | 63 | 35 | | | −34,0° | −145,5° | 0,2 | Dimethylsulfoxid/Methanol |
| Ddz-(1-6)-OBzl | 84% | farbl. amorph | 04 | 90 | 47 | | | −31,0° | −142,0° | 0,5 | $CH_2Cl_2$ |
| Ddz-Lys(Z)-(2-6)-OBzl | 100% | farbl. amorph | 16 | 79 | | | | — | — | — | — |

I: 7 : 1, II: 85 : 10 : 5, III: 93 : 7, IV: 4 : 1 : 1, V: sek.-Butanol/3%iger Ammoniak (100 : 44)

TABELLE VII

Thymosin$\alpha_1$-Fragmente I-IV und IX in Form der Peptidsäuren

| Fragment | | Ausbeute | Aussehen | Fp (°C) | hRf I | II | III | $[\alpha]^{20}$ 578 nm | 365 nm | c(g/100 ml) | Lösungsmittel |
|---|---|---|---|---|---|---|---|---|---|---|---|
| IV | Ddz-(20-24)-OH | 76% | glasartig | — | | 0 | 57 | | | | |
| III | Ddz-(13-19)-OH | 86% | weisses Pulver | 180-182 (Z) | 80[1] | 0 | 56 | −24,5° | −87,2° | 0,375 | Methanol |
| II | Ddz-(7-12)-OH | 57% | weisses Pulver | — | | 0 | 74 | −18,0° | −46,7° | 0,15 | Methanol |
| I | Ac-(1-6)-OH | 67% | weiss amorph | 219-221 (Z) | | 0 | 22 | −47,1° | −190,0° | 0,014 | 2,2,2-Trifluoräthanol |
| IX | Ac-(1-12)-OH | 85% | farbl. Glas | — | | 0 | 25[2] | −33,3° | −98,3° | 0,06 | |

I: 7 : 1, II: 93 : 7, III: 85 : 10 : 5, 1): 4 : 1 : 1, 2): $CHCl_3$ : 2,2,2-Trifluoräthanol/Essigsäure

TABELLE VIII

Thymosin$\alpha_1$-Fragmente VI-IX

| Fragment | | Ausbeute | Aussehen | Fp (°C) | hRf I | II | III | $[\alpha]^{20}$ 578 nm | 365 nm | c(g/100 ml) | Lösungsmittel |
|---|---|---|---|---|---|---|---|---|---|---|---|
| VI | Ddz-(20-28)-OBzl | 70% | glasartig | 252° (Z) | 25 | 72 | | +13,1° | +29,4° | 0,435 | $CHCl_3$ |
| VII | Ddz-(13-28)-OBzl | 60% | weisses Pulver | 260° (Z) | 00 | 60 | 88 | −8,1° | −49,8° | 0,235 | $CHCl_3$/Methanol (8 : 2) |
| VIII | Ddz-(7-27)-OBzl | 67% | glasartig | — | | 50 | | −9,7° | −25,2°[1] | 2,593 | 2,2,2-Trifluoräthanol |
| IX | Ac-(1-12)-OBzl | 61% | weisses Pulver | 263° (Z) | 34 | 77 | | −28,7° | −116,2° | 0,16 | 2,2,2-Trifluoräthanol |

I: 93 : 7, II: 85 : 10 : 5, III: 4 : 1 : 1, 1): 436 nm

TABELLE IX

Aminosäureanalyse des Thymosin$\alpha_1$-Fragments IV

| Fragment | | Hydrolyse-methode | Asp | Glu | Ala | Val | Lys |
|---|---|---|---|---|---|---|---|
| IV | Ddz-(20-24)-OME | a | | 1,82 (2) | | 2,00 (2) | 1,05 (1) |

Hydrolysemethode: a = HCl/Propionsäure 160°C 45 bis 60 Minuten

TABELLE X

Aminosäureanalyse der Thymosin$\alpha_1$-Fragmente I bis III

| Frag-ment | | Hydro lyse-methode | Asp | Thr | Ser | Glu | Ala | Val | Ile | Leu | Lys |
|---|---|---|---|---|---|---|---|---|---|---|---|
| III | Ddz-(13-19)-OMe | a | 0,96 (1) | 0,6 (1) | | 1,05 (1) | | | | 1,00 (1) | 2,75 (3) |
| II | Ddz-(7-12)-OBzl | b | | 1,82 (2) | 1,96 (2) | 1,08 (1) | | | 1,00 (1) | | |
| I | Ac-(1-6)-OBzl | b | 1,97 (2) | | 0,99 (1) | | 1,95 (2) | 1,00 (1) | | | |

Hydrolysemethode: a = HCl/Propionsäure 160°C, 45 bis 60 Minuten
b = 6 n HCl 110°C, 18 bis 24 Stunden

TABELLE XI

Aminosäureanalyse der Thymosin$\alpha_1$-Fragmente I bis IX

| Frag-ment | | Hydro-lyse-methode | Asp | Thr | Ser | Glu | Ala | Val | Ile | Leu | Lys |
|---|---|---|---|---|---|---|---|---|---|---|---|
| IV | Ddz-(20-24)-OH | a | | | | 1,82 (2) | | 2,00 (2) | | | 1,05 (1) |
| VI | Ddz-(20-28)-OBzl | b | 1,70 (1) | | | 4,3 (4) | 1,00 (1) | 1,36 (2) | | | 1,00 (1) |
| III | Ddz-(13-19)-OH | b | 1,12 (1) | 0,85 (1) | | 1,02 (1) | | | | 1,00 (1) | 2,75 (3) |
| VII | Ddz-(13-28)-OBzl | b | 1,94 (2) | 0,7 (1) | | 4,44 (5) | 0,71 (1) | 1,44 (1) | | 1,00 (1) | 3,76 (4) |
| II | Ddz-(7-12)-OMe | a | | 1,52 (2) | 1,38 (2) | 0,77 (1) | | | 1,00 (1) | | |
| II | Ddz-(7-12)-OH | b | | 1,75 (2) | 1,78 (1) | 1,16 (1) | | | 1,00 (1) | | |
| VIII | Ddz-(7-28)-OBzl | a | 2,09 (2) | 2,56 (3) | 1,07 (2) | 5,22 (6) | 0,78 (1) | 1,44 (2) | 0,85 (1) | 1,15 (1) | 3,91 (4) |
| I | Ac-(1-6)-OH | b | 1,84 (2) | | 0,91 (1) | | 1,97 (2) | 1,00 (1) | | | |
| IX | Ac-(1-12)-OBzl | b | 2,08 (2) | 1,87 (2) | 2,5 (3) | 0,56 (1) | 2,42 (2) | 1,00 (1) | 0,96 (1) | | |
| IX | Ac-(1-12)-OH | b | 2,12 (2) | 1,77 (2) | 2,45 (3) | 1,00 (1) | 2,12 (2) | 1,00 (1) | 1,10 (1) | | |
| Ia | Ddz-Lys-(2-6)-OBzl | a | 2,10 (2) | | | | 1,92 (2) | 1,00 (1) | | | 1,03 (1) |
| IVa | Ddz-(20-23)-Gla-OMe | a | | | | 2,11 (2) | | 2,00 (2) | | | 1,02 (1) |
| Va | Ddz-Gla-(25-28)-OBzl | a | 0,87 (1) | | | 2,09 (2) | 1,00 (1) | | | | |
| VIa | Ddz-(20-23-$Gla_2$-25-28)-OBzl | a | 1,11 (1) | | | 4,23 (4) | 1,00 (1) | 1,79 (2) | | | 0,98 (1) |

Hydrolysemethode: a = HCl/Propionsäure 160°C, 45 bis 60 Minuten
b = 6 n HCl 110°C, 18 bis 24 Stunden

*Beispiel 9*

Zum Nachweis der immunstimulierenden bzw. immunregulierenden Eigenschaften der erfindungsgemässen Thymosin$\alpha_1$-Fragmente wurden immunologisch-pharmakologische Untersuchungen durchgeführt.

Als immunologische Testmethoden wurden dabei der E-Rosettentest nach Wara, Ammann et al. (N.Y. Acad. Sci. 249 (1975) 308 und N. Engl. J. Med. 292 (1975) 70) und die Methode der gemischten Lymphozytenkultur angewandt, beide mit zusätzlicher Inhibierung der zellulären RNS-Polymerase durch $\alpha$-Amanitin.

Der E-Rosettentest beruht darauf, dass der Prozentsatz von E-rosettenbildenden Zellen im peripheren menschlichen Blut ein Mass für den Gehalt an ausgereiften T-Zellen darstellt, die mit roten Schafsblutzellen charakteristische Aggregate bilden, die unter dem Mikroskop ausgezählt werden können. Untersuchungen von Wara, Ammann et al. (loc. cit.) haben ergeben, dass bei Patienten mit Thymushypoplasie eine Steigerung von 20% auf 45% der E-Rosettenbildung erreicht werden kann, vorausgesetzt, es wird ein optimal aktives Thymosin-Präparat zugesetzt. Der Normalwert der E-Rosettenbildung beim gesunden Menschen liegt bei 56%.

Zur Durchführung des E-Rosettentests werden periphere menschliche Lymphozyten durch eine Gradientenzentrifugation isoliert. Ihre RNS-Polymerase wird durch $\alpha$-Amanitin blockiert. Dann werden rote Schafsblutzellen und Thymosin oder ein Thymosin$\alpha_1$-Fragment hinzugefügt, das die $\alpha$-Amanitinblockade der E-Rosettenbildung aufhebt, so dass der Normalwert der Zellaggregat-Bildung von 56% erreicht werden kann. In einer relativen Zahlenskala wird dieser Wert zu 100%, der nach $\alpha$-Amanitinblockade zu 0% gesetzt, siehe Tabelle XII.

Der Test in gemischten Lymphozytenkulturen beruht darauf, dass T-Zellen durch Stimulierung mit allogenen Antigenen zur Vermehrung angeregt werden. Zur Durchführung des Tests werden die Lymphozyten des Spenders A (Responder) mit denen des Spenders B (Stimulator) während 4 bis 5 Tagen inkubiert. Dann wird $^3$H-Thymidin zugesetzt und anhand zunehmender Radioaktivität der Zellkultur dank Einbau des radioaktiven Materials die Vermehrungsrate gemessen. Da beide Populationen A und B zur Vermehrung angeregt werden könnten und so einen unklaren Effekt ergäben, wird die Population B mit Mitomycin C in allen Stoffwechselaktivitäten blockiert. Mit diesem Test wird routinemässig vor Organtransplantationen die immunologische Verträglichkeit von Spender- und Empfängergewebe festgestellt.

Die bei diesen pharmakologischen Untersuchungen erzielten Ergebnisse sind in den nachstehenden Tabellen XII und XIII zusammengestellt.

TABELLE XII

Beispiele der Stimulation von menschlichen peripheren Lymphozyten mit Thymosin$\alpha_1$-Fragmenten im E-Rosettentest nach Blockierung der Zellen mit $\alpha$-Amanitin (0%-Wert der E-Rosettenzahl; Normalwert ohne jeden Zusatz = 100%)

| Fragment | Ia | II | III | IV | VI | VII | IX | Thymosin$\alpha_1$ (synth.) |
|---|---|---|---|---|---|---|---|---|
| E-Rosettenzahl (%) | 93 | 29 | 29 | 45 | 16 | 41 | 40 | 100 |
| Optimale Konzentration ($\mu$M) | 16 | 80 | 80 | 83 | 10 | 6 | 4 | 1,6 |

TABELLE XIII

Beispiele der Stimulation von menschlichen peripheren Lymphozyten durch Thymosin$\alpha_1$-Fragmente im Test mit gemischten Lymphozytenkulturen, ohne und mit Blockierung durch $\alpha$-Amanitin

(1 $\mu$g Thymosin$\alpha_1$-Fragment/ml Zellkultur; 1 $\mu$g $\alpha$-Amanitin/ml Zellkultur (+) = Stimulation (—) = Inhibition (%) der Kultur durch Thymosin$\alpha_1$-Fragmentzusatz. Die Reaktion der gemischten Lymphozytenkultur ohne diesen Zusatz wird zu 0% gesetzt)

| Fragment | | Ia | II | III | IV | VI | IX |
|---|---|---|---|---|---|---|---|
| Zellpaare | AB | − 8 | — | −64 | − 53 | + 153 | −74 |
| | CD | −73 | −53 | −13 | + 37 | + 57 | −63 |
| | BA | −15 | −18 | −12 | +565 | +1136 | — |
| | DC | −31 | +30 | +12 | + 2 | ± 0 | −77 |
| Zellpaare$^+$ | AB | −10 | −11 | −44 | − 87 | + 279 | −30 |
| | CD | −53 | −98 | −20 | + 35 | + 83 | +63 |
| | BA | — | −80 | + 6 | − 97 | − 96 | −78 |
| | DC | − 7 | +20 | +80 | +100 | + 160 | +60 |

+) Kulturen blockiert mit $\alpha$-Amanitin

Aus den obigen Tabellen XII und XIII ist ohne weiteres ersichtlich, dass die erfindungsgemässen Thymosin$\alpha_1$-Fragmente, wie anhand der Beispiele in den Tabellen XII und XIII gezeigt, eine sehr ausgeprägte immunologische Wirkung entfalten, die ihre Anwendung in der Humantherapie und der Veterinärmedizin ermöglicht.

Vergleicht man die in den obigen Beispielen gezeigten immunologischen Aktivitäten der Fragmente zunächst allgemein mit denen der Muttersubstanz, totalsynthetischem Thymosin$\alpha_1$ (dargestellt in der Tabelle XIV für den E-Rosettentest und in Tabelle XV für die gemischte Lymphozytenkultur), so lässt sich ableiten, dass sämtliche untersuchten Fragmente sowohl in den Differenzierungsmechanismus und die Reifung von Thymus-abhängigen Lymphozyten zu immunkompetenten T-Zellen als auch in deren Vermehrungsprozess (Proliferation) ausgeprägt eingreifen.

Die Vergleiche im Detail der immunologischen Aktivitäten der Fragmente mit Thymosin$\alpha_1$ zeigen deutlich, dass Mechanismen der T-Zellenaktivierung, die im E-Rosettentest zum Ausdruck kommen, von N-terminalen Thymosin$\alpha_1$-Fragmenten (VII, IX) stärker angeregt werden als von den C-terminalen Teilstücken IV und VI. Eine Sonderstellung nimmt hier das besonders aktive unnatürliche Fragment Ia ein, das mit seiner zusätzlichen basischen Funktion (Lysin) offensichtlich einen Effekt an T-Lymphozyten ausübt, der bei den natürlichen Fragmenten erst in Kombination mit mittleren Sequenzabschnitten bewirkt wird. Diese im E-Rosettentest besonders aktiven Fragmente zeigen in der gemischten Lymphozytenkultur ebenfalls eine überraschend ausgeprägte inhibitorische Wirkung (siehe Tabelle XIII für Fragment Ia und IX) auf die gegenseitige zellgebundene Antigenität von Lymphozyten verschiedener Spender aufeinander. Dieses Ergebnis kann für die Transplantationsmedizin beträchtliche Bedeutung erlangen durch die mögliche Herstellung von Arzneimitteln mit immunsuppressorischer Wirkung auf der Basis kleiner, einfach zu synthetisierender Peptide.

Anhand der Vergleiche von Thymosin$\alpha_1$ mit den Fragmenten IV und VI kann jedoch aus beiden Bioassays abgeleitet werden, dass das immunologisch stimulative Zentrum von Thymosin$\alpha$ im C-terminalen Bereich lokalisiert ist. Hierbei muss besonders auf das einfach zu synthetisierende Peptid Lys-Glu-Val-Val-Glu (Fragment IV) hingewiesen werden, das zwar erst in höherer Dosis im E-Rosettentest Aktivität zeigt, aber eine ganz besonders beachtliche Stimulation der T-Zellenproliferation in der gemischten Lymphozytenkultur entfaltet.

Bei einem Mangel an immunkompetenten T-Zellen (T-Lymphozyten) ist nämlich vor allem die immunologische Erkennung (zellgebundene Antikörperbildung) und Abwehr entartender Körperzellen herabgesetzt. Zelluläre Entartungen können bedingt sein durch Virusinfektionen, biologische Alterungsprozesse (zelluläre Fehlsteuerung) und verschiedene Entstehungsformen von Krebs.

Die vorgelegten Ergebnisse lassen erwarten, dass solche Fragmente des Thymosin$\alpha_1$, die der Muttersubstanz äquivalent oder potentere Aktivitäten zeigen, für die Herstellung von Arzneimitteln gegen immunologische Mangelkrankheiten und Altersschwächen, Virusinfektionen, vor allem aber gegen verschiedene Formen des Krebses (Leukämie, Lungen-Metastasen) besonders geeignet sein sollten.

TABELLE XIV

Vergleichsdaten synthetischen Thymosin$\alpha_1$ mit einem natürlichen Polypeptid aus Thymus im E-Rosettentest mit $\alpha$-Amanitin-Inhibition

Normale Zellpopulation: 64%[a] E-Rosetten
$\alpha$-Amanitin inhibiert: 25%[a] E-Rosetten

| Thymus-Polypeptid[b] | | Synthetisches Thymosin$\alpha_1$[c] | |
|---|---|---|---|
| $\mu$g/Kultur | (%)[d] | $\mu$g/Kultur | (%)[d] |
| 6,25 | 10 | 5,00 | 33 |
| 3,12 | 67 | 2,50 | 57 |
| 1,25 | 100 | 1,00 | 64 |
| 0,62 | 62 | 0,50 | 100 |
| 0,31 | 6 | 0,25 | 39 |

a) Absolut-Wert
b) Aus Thymus isoliert
c) Analytisch rein
d) Relative Anzahl der durch Stimulation mit Thymus-Peptiden wieder erzeugten E-Rosetten nach deren vorheriger Inhibierung mit $\alpha$-Amanitin, aus peripheren menschlichen Lymphozyten.

TABELLE XV

Die Stimulation durch synthetisches Thymosin$\alpha_1$ einer allogenen gemischten Kultur aus peripheren humanen Lymphozyten (Stimulatorzellen Mitomycinblockiert)

| Zellpaare | (%) a) | Stimulation[c] b) |
|---|---|---|
| AB | + 20 | + 86 |
| AD | + 4 | + 37 |
| DB | + 27 | + 1550 |
| BA | + 27 | + 47 |
| BD | + 45 | + 51 |
| DA | + 44 | ± 0 |

a) nicht inhibierte Kultur
b) Kultur inhibiert mit 1 $\mu$g $\alpha$-Amanitin/ml Zellsuspension
c) Kulturen stimuliert durch Zusatz von 1 $\mu$g Thymosin$\alpha_1$/ml Zellsuspension; die Lymphozytenstimulation in der Kultur ohne Zusatz von Thymosin$\alpha_1$ wurde zu 0% gesetzt.

**Patentansprüche**

1. Arzneimittel mit immunstimulierender Wirkung, gekennzeichnet durch den Gehalt mindestens eines Thymosin$\alpha_1$-Fragments und/oder mindestens eines Derivats davon in freier Form oder in Form eines pharmakologisch annehmbaren Salzes, ausgenommen solche der allgemeinen Formel X-Glu-Asn-OH, worin X die Bedeutung H, H-Ala-, H-Glu-Ala-, H-Glu-Glu-Ala-, H-Val-Glu-Glu-Ala- oder H-Val-Val-Glu-Glu-Ala- bedeutet und deren physiologisch verträgliche Salze.

2. Arzneimittel nach Anspruch 1, dadurch gekennzeichnet, dass es als Derivat des Thymosin$\alpha_1$-Fragments ein Thymosin$\alpha_1$-Fragment enthält, dessen N-Terminus in freier Form vorliegt oder eine Acylgruppe mit 1 bis 6 Kohlenstoffatomen, vorzugsweise eine Acetylgruppe oder eine Acylglycingruppe trägt, deren Acylgruppe 1 bis 6 Kohlenstoffatome aufweist.

3. Arzneimittel nach den Ansprüchen 1 oder 2, dadurch gekennzeichnet, dass es das Fragment I der Formel

1 2 3 4 5 6
Ser-Asp-Ala-Ala-Val-Asp

enthält.

4. Arzneimittel nach den Ansprüchen 1 oder 2, dadurch gekennzeichnet, dass es das in Position 1 durch einen Lysin-Rest abgewandelte Fragment Ia enthält der Formel

1 2 3 4 5 6
Lys-Asp-Ala-Ala-Val-Asp

5. Arzneimittel nach den Ansprüchen 1 oder 2, dadurch gekennzeichnet, dass es das Fragment II der Formel

7 8 9 10 11 12
Thr-Ser-Ser-Glu-Ile-Thr

enthält.

6. Arzneimittel nach Anspruch 5, dadurch gekennzeichnet, dass es das Fragment II

7 8 9 10 11 12
Thr-Ser-Ser-Glu-Ile-Thr

enthält, dessen Glutaminsäurerest 10 als Amid oder Alkylamid mit 1 bis 6 Kohlenstoffatomen in der Alkylgruppe vorliegt.

7. Arzneimittel nach den Ansprüchen 1 oder 2, dadurch gekennzeichnet, dass es das Fragment III der Formel

13 14 15 16 17 18 19
Thr-Lys-Asp-Leu-Lys-Glu-Lys

enthält.

8. Arzneimittel nach Anspruch 7, dadurch gekennzeichnet, dass es das Fragment III der Formel

13 14 15 16 17 18 19
Thr-Lys-Asp-Leu-Lys-Glu-Lys

enthält, dessen Asparaginsäurerest 15 als Amid oder Alkylamid mit 1 bis 6 Kohlenstoffatomen in der Alkylgruppe vorliegt.

9. Arzneimittel nach den Ansprüchen 1 oder 2, dadurch gekennzeichnet, dass es das Fragment IV der Formel

20 21 22 23 24
Lys-Glu-Val-Val-Glu

enthält.

10. Arzneimittel nach den Ansprüchen 1 oder 2, dadurch gekennzeichnet, dass es das in Position 24 durch $\gamma$-Carboxyglutaminsäure-(Gla)-Rest abgewandelte Fragment IVa der Formel

20 21 22 23 24
Lys-Glu-Val-Val-Gla

enthält.

11. Arzneimittel nach Anspruch 10, dadurch gekennzeichnet, dass es das Fragment IV der Formel

20 21 22 23 24
Lys-Glu-Val-Val-Glu

enthält, in der der Glutaminsäurerest 21 und/oder 24 als Amid oder Alkylamid mit 1 bis 6 Kohlenstoffatomen in der Alkylgruppe vorliegt.

12. Arzneimittel nach den Ansprüchen 1 oder 2, dadurch gekennzeichnet, dass es das in Position 25 durch den Gla-Rest abgewandelte Fragment Va der Formel

25 26 27 28
Gla-Ala-Glu-Asn

enthält.

13. Arzneimittel nach Anspruch 12, dadurch gekennzeichnet, dass es das Fragment V der Formel

25 26 27 28
Glu-Ala-Glu-Asn

enthält, dessen Glutaminsäurerest 25 und/oder 27 sowie dessen Asparaginrest 28 als Amid oder Alkylamid bzw. als Diamid oder Dialkylamid, wobei die Alkylgruppen 1 bis 6 Kohlenstoffatome aufweisen, vorliegt.

14. Arzneimittel nach den Ansprüchen 1 oder 2, dadurch gekennzeichnet, dass es das Fragment VI der Formel

20 21 22 23 24 25 26 27 28
Lys-Glu-Val-Val-Glu-Glu-Ala-Glu-Asn

enthält.

15. Arzneimittel nach Anspruch 14, dadurch gekennzeichnet, dass es das Fragment VI der Formel

20 21 22 23 24 25 26 27 28
Lys-Glu-Val-Val-Glu-Glu-Ala-Glu-Asn

enthält, dessen Glutaminsäurereste 21 und 25 und/oder dessen Asparaginrest 28 als Amid oder Alkylamid bzw. als Diamid oder Dialkylamid, wobei die Alkylgruppen 1 bis 6 Kohlenstoffatome aufweisen, vorliegt.

16. Arzneimittel nach den Ansprüchen 1 oder 2, dadurch gekennzeichnet, dass es das Fragment VII der Formel

13 14 15 16 17 18 19 20 21 22 23 24
Thr-Lys-Asp-Leu-Lys-Glu-Lys-Lys-Glu-Val-Val-Glu-
25 26 27 28
Glu-Ala-Glu-Asn

enthält.

17. Arzneimittel nach Anspruch 16, dadurch gekennzeichnet, dass es das Fragment VII der Formel

13 14 15 16 17 18 19 20 21 22 23 24
Thr-Lys-Asp-Leu-Lys-Glu-Lys-Lys-Glu-Val-Val-Glu-
25 26 27 28
Glu-Ala-Glu-Asn

enthält, dessen Asparaginsäurerest 15 und/oder dessen Glutaminsäurerest 21, 24, 25 und/oder 27 sowie dessen Asparaginrest 28 als Amid oder Alkylamid bzw. Diamid oder Dialkylamid mit 1 bis 6 Kohlenstoffatomen in der Alkylgruppe vorliegt.

18. Arzneimittel nach den Ansprüchen 1 oder 2, dadurch gekennzeichnet, dass es das Fragment VIII der Formel

7 8 9 10 11 12 13 14 15 16 17 18
Thr-Ser-Ser-Glu-Ile-Thr-Thr-Lys-Asp-Leu-Lys-Glu-
19 20 21 22 23 24 25 26 27 28
Lys-Lys-Glu-Val-Val-Glu-Glu-Ala-Glu-Asn

enthält.

19. Arzneimittel nach Anspruch 18, dadurch gekennzeichnet, dass es das Fragment VIII der Formel

7 8 9 10 11 12 13 14 15 16 17 18
Thr-Ser-Ser-Glu-Ile-Thr-Thr-Lys-Asp-Leu-Lys-Glu-
19 20 21 22 23 24 25 26 27 28
Lys-Lys-Glu-Val-Val-Glu-Glu-Ala-Glu-Asn

enthält, dessen Glutaminsäurereste 10, 21, 24, 25 und 27, dessen Asparaginsäurerest 15 und/oder dessen Asparaginrest 28 als Amid oder Alkylamid bzw. Diamid oder Dialkylamid, wobei die Alkylgruppen 1 bis 6 Kohlenstoffatome aufweisen, vorliegt.

20. Arzneimittel nach den Ansprüchen 1 oder 2, dadurch gekennzeichnet, dass es das Fragment IX der Formel

1 2 3 4 5 6 7 8 9 10 11 12
Ser-Asp-Ala-Ala-Val-Asp-Thr-Ser-Ser-Glu-Ile-Thr

enthält.

21. Arzneimittel nach Anspruch 20, dadurch gekennzeichnet, dass es das Fragment IX der Formel

1 2 3 4 5 6 7 8 9 10 11 12
Ser-Asp-Ala-Ala-Val-Asp-Thr-Ser-Ser-Glu-Ile-Thr

enthält, dessen Glutaminsäurerest 10 als Amid bzw. Alkylamid mit 1 bis 6 Kohlenstoffatomen in der Alkylgruppe vorliegt.

22. Arzneimittel nach mindestens einem der vorhergehenden Ansprüche, dadurch gekennzeichnet, dass es neben dem oder den Wirkstoffen übliche, für den gewählten Verabreichungsweg geeignete, pharmakologisch annehmbare Bindemittel, Trägermaterialien und/oder Hilfsstoffe enthält.

23. Thymosin$\alpha_1$-Fragment I der Formel

1 2 3 4 5 6
Ser-Asp-Ala-Ala-Val-Asp

wobei das Fragment N-terminal eine Acylgruppe mit 1 bis 6 Kohlenstoffatomen, insbesondere eine Acetylgruppe oder eine Acylglycingruppe, deren Acylgruppe 1 bis 6 Kohlenstoffatome aufweist, tragen kann, sowie die pharmazeutisch annehmbaren Salze dieses Fragments.

24. Thymosin$\alpha_1$-Fragment II der Formel

7 8 9 10 11 12
Thr-Ser-Ser-Glu-Ile-Thr

wobei das Fragment N-terminal eine Acylgruppe mit 1 bis 6 Kohlenstoffatomen, insbesondere eine Acetylgruppe oder eine Acylglycingruppe, deren Acylgruppe 1 bis 6 Kohlenstoffatome aufweist, tragen kann und der Glutaminsäurerest 10 als Amid oder Alkylamid mit 1 bis 6 Kohlenstoffatomen in der Alkylgruppe vorliegen kann, sowie die pharmazeutisch annehmbaren Salze dieses Fragments.

25. Thymosin$\alpha_1$-Fragment III der Formel

13 14 15 16 17 18 19
Thr-Lys-Asp-Leu-Lys-Glu-Lys

wobei das Fragment N-terminal eine Acylgruppe mit 1 bis 6 Kohlenstoffatomen, insbesondere eine Acetylgruppe oder eine Acylglycingruppe, deren Acylgruppe 1 bis 6 Kohlenstoffatome aufweist, tragen kann und der Asparaginsäurerest 15 als Amid oder Alkylamid mit 1 bis 6 Kohlenstoffatomen in der Alkylgruppe vorliegen kann, sowie die pharmazeutisch annehmbaren Salze dieses Fragments.

26. Thymosin$\alpha_1$-Fragment IV der Formel

20 21 22 23 24
Lys-Glu-Val-Val-Glu

wobei das Fragment N-terminal eine Acylgruppe mit 1 bis 6 Kohlenstoffatomen, insbesondere eine Acetylgruppe oder eine Acylglycingruppe, deren Acylgruppe 1 bis 6 Kohlenstoffatome aufweist, tragen kann und der Glutaminsäurerest 21 und 24 als Amid oder Alkylamid mit 1 bis 6 Kohlenstoffatomen in der Alkylgruppe vorliegen kann, sowie die pharmazeutisch annehmbaren Salze dieses Fragments.

27. Thymosin$\alpha_1$-Fragment V der Formel

25 26 27 28
Glu-Ala-Glu-Asn

welches N-terminal eine Acylgruppe mit 1 bis 6 Kohlenstoffatomen, insbesondere eine Acetylgruppe oder eine Acylglycingruppe, deren Acylgruppe 1 bis 6 Kohlenstoffatome aufweist, trägt und/oder der Glutaminsäurerest 25 und/oder 27 als Amid oder Alkylamid sowie dessen Asparaginrest 28 als Diamid

oder Dialkylamid mit 1 bis 6 Kohlenstoffatomen in der Alkylgruppe vorliegen kann, sowie die pharmazeutisch annehmbaren Salze dieser Derivate.

28. Thymosin$\alpha_1$-Fragment VI der Formel

20 21 22 23 24 25 26 27 28
Lys-Glu-Val-Val-Glu-Glu-Ala-Glu-Asn

wobei das Fragment N-terminal eine Acylgruppe mit 1 bis 6 Kohlenstoffatomen, insbesondere eine Acetylgruppe oder eine Acylglycingruppe mit 1 bis 6 Kohlenstoffatomen in der Acylgruppe tragen kann und die Glutaminsäurereste 21, 24, 25 und/oder 27 sowie der Asparaginrest 28 als Amid oder Alkylamid bzw. als Diamid oder als Dialkylamid mit 1 bis 6 Kohlenstoffatomen in den Alkylgruppen vorliegen können, sowie die pharmazeutisch annehmbaren Salze dieses Fragments.

29. Thymosin$\alpha_1$-Fragment VII der Formel

13 14 15 16 17 18 19 20 21 22 23 24
Thr-Lys-Asp-Leu-Lys-Glu-Lys-Lys-Glu-Val-Val-Glu-
25 26 27 28
Glu-Ala-Glu-Asn

wobei das Fragment N-terminal eine Acylgruppe mit 1 bis 6 Kohlenstoffatomen, insbesondere eine Acetylgruppe oder eine Acylglycingruppe, deren Acylgruppe 1 bis 6 Kohlenstoffatome aufweist, tragen kann und die Glutaminsäurereste 21, 24, 25 und/oder 27, der Asparaginsäurerest 15 sowie der Asparaginrest 28 als Amid oder Alkylamid bzw. als Diamid oder als Dialkylamid mit 1 bis 6 Kohlenstoffatomen in den Alyklgruppen vorliegen können, sowie die pharmazeutisch annehmbaren Salze dieses Fragments.

30. Thymosin$\alpha_1$-Fragment VIII der Formel

7 8 9 10 11 12 13 14 15 16 17 18
Thr-Ser-Ser-Glu-Ile-Thr-Thr-Lys-Asp-Leu-Lys-Glu-
19 20 21 22 23 24 25 26 27 28
Lys-Lys-Glu-Val-Val-Glu-Glu-Ala-Glu-Asn

wobei das Fragment N-terminal eine Acylgruppe mit 1 bis 6 Kohlenstoffatomen, insbesondere eine Acetylgruppe oder eine Acylglycingruppe, deren Acylgruppe 1 bis 6 Kohlenstoffatome aufweist, tragen kann und die Glutaminsäurereste 10, 21, 24, 25 und/oder 27, der Asparaginsäurerest 15 sowie der Asparaginrest 28 als Amid oder Alkylamid bzw. als Diamid oder als Dialkylamid mit 1 bis 6 Kohlenstoffatomen in den Alkylgruppen vorliegen können, sowie die pharmazeutisch annehmbaren Salze dieses Fragments.

31. Thymosin$\alpha_1$-Fragment IX der Formel

1 2 3 4 5 6 7 8 9 10 11 12
Ser-Asp-Ala-Ala-Val-Asp-Thr-Ser-Ser-Glu-Ile-Thr

wobei das Fragment N-terminal eine Acylgruppe mit 1 bis 6 Kohlenstoffatomen, insbesondere eine Acetylgruppe oder eine Acylglycingruppe, deren Acylgruppe 1 bis 6 Kohlenstoffatome aufweist, tragen kann und der Glutaminsäurerest 10 als Amid oder Alkylamid mit 1 bis 6 Kohlenstoffatomen in der Alkylgruppe vorliegen kann, sowie die pharmazeutisch annehmbaren Salze dieses Fragments.

32. Arzneimittel nach den Ansprüchen 1 oder 2, dadurch gekennzeichnet, dass es das in Position 24 und 25 durch Gla-Reste abgewandelte Fragment VIa der Formel

20 21 22 23 24 25 26 27 28
Lys-Glu-Val-Val-Gla-Gla-Ala-Glu-Asn (VIa)

enthält.

33. Thymosin$\alpha_1$-Fragment Ia der Formel

1 2 3 4 5 6
Lys-Asp-Ala-Ala-Val-Asp (Ia)

wobei das Fragment N-terminal eine Acylgruppe mit 1 bis 6 Kohlenstoffatomen, insbesondere eine Acetylgruppe oder eine Acylglycingruppe, deren Acylgruppe 1 bis 6 Kohlenstoffatome aufweist, tragen kann, sowie die pharmazeutisch annehmbaren Salze dieses Fragments.

34. Thymosin$\alpha_1$-Fragment IVa der Formel

20 21 22 23 24
Lys-Glu-Val-Val-Gla (IVa)

wobei das Fragment N-terminal eine Acylgruppe mit 1 bis 6 Kohlenstoffatomen, insbesondere eine Acetylgruppe oder eine Acylglycingruppe, deren Acylgruppe 1 bis 6 Kohlenstoffatome aufweist, tragen kann und der Glutaminsäurerest 21 als Amid oder Alkylamid mit 1 bis 6 Kohlenstoffatomen in der Alkylgruppe vorliegen kann, sowie die pharmazeutisch annehmbaren Salze dieses Fragments.

35. Thymosin$\alpha_1$-Fragment Va der Formel

25 26 27 28
Gla-Ala-Glu-Asn (Va)

wobei das Fragment N-terminal eine Acylgruppe mit 1 bis 6 Kohlenstoffatomen, insbesondere eine Acetylgruppe oder eine Acylglycingruppe, deren Acylgruppe 1 bis 6 Kohlenstoffatome aufweist, tragen kann und der Glutaminsäurerest 27 als Amid oder Alkylamid sowie dessen Asparaginrest 28 als Diamid oder Dialkylamid mit 1 bis 6 Kohlenstoffatomen in der Alkylgruppe vorliegen kann, sowie die pharmazeutisch annehmbaren Salze dieses Fragments.

36. Thymosin$\alpha_1$-Fragment VIa der Formel

20 21 22 23 24 25 26 27 28
Lys-Glu-Val-Val-Gla-Gla-Ala-Glu-Asn (VIa)

wobei das Fragment N-terminal eine Acylgruppe mit 1 bis 6 Kohlenstoffatomen, insbesondere eine Acetylgruppe oder eine Acylglycingruppe mit 1 bis 6 Kohlenstoffatomen in der Acylgruppe tragen kann und die Glutaminsäurereste 21 und/oder 27 sowie der Asparaginrest 28 als Amid oder Alkylamid bzw. als Diamid oder als Dialkylamid mit 1 bis 6 Kohlenstoffatomen in der Alkylgruppe vorliegen können, sowie die pharmazeutisch annehmbaren Salze dieses Fragments.

**Claims**

1. Medicament with immunostimulant activity characterised by a content of at least one thymosin-$\alpha_1$-fragment and/or at least one derivative thereof in free form or in the form of a pharmacologically acceptable salt, with the exception of those of the general formula X-Glu-Asn-OH, wherein X has the meaning H, H-Ala-, H-Glu-Ala-, H-Glu-Glu-Ala-, H-Val-Glu-Glu-Ala- or H-Val-Val-Glu-Glu-Ala-, and the physiologically compatible salts thereof.

2. Medicament according to claim 1, characterised in that as a derivative of the thymosin$\alpha_1$-fragment it contains a thymosin$\alpha_1$-fragment whose N-terminal group is present in free form or carries an acyl group with 1 to 6 carbon atoms, preferably an acetyl group or an acylglycine group, the acyl group of which has 1 to 6 carbon atoms.

3. Medicament according to claims 1 or 2, characterised in that it contains the fragment I of the formula

1 2 3 4 5 6
Ser-Asp-Ala-Ala-Val-Asp

4. Medicament according to claims 1 or 2, characterised in that it contains the fragment Ia, modified in position I by a lysine residue, of the formula

1 2 3 4 5 6
Lys-Asp-Ala-Ala-Val-Asp

5. Medicament according to claims 1 or 2, characterised in that it contains the fragment II of the formula

7 8 9 10 11 12
Thr-Ser-Ser-Glu-Ile-Thr

6. Medicament according to claim 5, characterised in that it contains the fragment II

7 8 9 10 11 12
Thr-Ser-Ser-Glu-Ile-Thr

whose glutamic acid residue 10 is present as an amide or an alkylamide with 1 to 6 carbon atoms in the alkyl group.

7. Medicament according to claims 1 or 2, characterised in that it contains the fragment III of the formula

13 14 15 16 17 18 19
Thr-Lys-Asp-Leu-Lys-Glu-Lys

8. Medicament according to claim 7, characterised in that it contains the fragment III of the formula

13 14 15 16 17 18 19
Thr-Lys-Asp-Leu-Lys-Glu-Lys

whose asparagic acid residue 15 is present as an amide or an alkylamide with 1 to 6 carbon atoms in the alkyl group.

9. Medicament according to claims 1 or 2, characterised in that it contains the fragment IV of the formula

20 21 22 23 24
Lys-Glu-Val-Val-Glu

10. Medicament according to claims 1 or 2, characterised in that it contains the fragment IVa, modified in position 24 by $\gamma$-carboxy glutamic acid-(Gla)-residue, of the formula

20 21 22 23 24
Lys-Glu-Val-Val-Gla

11. Medicament according to claim 10, characterised in that it contains the fragment IV of the formula

20 21 22 23 24
Lys-Glu-Val-Val-Glu

in which the glutamic acid residue 21 and/or 24 is present as an amide or an alkylamide with 1 to 6 carbon atoms in the alkyl group.

12. Medicament according to claims 1 or 2, characterised in that it contains the fragment Va, modified in position 25 by the Gla-residue, of the formula

25 26 27 28
Gla-Ala-Glu-Asn

13. Medicament according to claim 12, characterised in that it contains the fragment V of the formula

25 26 27 28
Glu-Ala-Glu-Asn

whose glutamic acid residue 25 and/or 27 and also whose asparagine residue 28 is present as an amide or an alkylamide and as a diamide or a dialkylamide, respectively, the alkyl groups having 1 to 6 carbon atoms.

14. Medicament according to claims 1 or 2, characterised in that it contains the fragment VI of the formula

20 21 22 23 24 25 26 27 28
Lys-Glu-Val-Val-Glu-Glu-Ala-Glu-Asn

15. Medicament according to claim 14, characterised in that it contains the fragment VI of the formula

20 21 22 23 24 25 26 27 28
Lys-Glu-Val-Val-Glu-Glu-Ala-Glu-Asn

whose glutamic acid residues 21 and 25 and/or whose asparagine residue 28 is present as an amide or an alkylamide and as a diamide or a dialkylamide, respectively, the alkyl groups having 1 to 6 carbon atoms.

16. Medicament according to claims 1 or 2, characterised in that it contains the fragment VII of the formula

13 14 15 16 17 18 19 20 21 22 23 24
Thr-Lys-Asp-Leu-Lys-Glu-Lys-Lys-Glu-Val-Val-Glu-
25 26 27 28
Glu-Ala-Glu-Asn

17. Medicament according to claim 16, characterised in that it contains the fragment of the formula

13 14 15 16 17 18 19 20 21 22 23 24
Thr-Lys-Asp-Leu-Lys-Glu-Lys-Lys-Glu-Val-Val-Glu-
25 26 27 28
Glu-Ala-Glu-Asn

whose asparagic acid residue 15 and/or whose glutamic acid residue 21, 24, 25 and/or 27 and also whose asparagine residue 28 is present as an amide or an alkylamide and as a diamide or a dialkylamide, respectively, with 1 to 6 carbon atoms in the alkyl group.

18. Medicament according to claims 1 to 2, characterised in that it contains the fragment VIII of the formula

7 8 9 10 11 12 13 14 15 16 17 18
Thr-Ser-Ser-Glu-Ile-Thr-Thr-Lys-Asp-Leu-Lys-Glu-
19 20 21 22 23 24 25 26 27 28
Lys-Lys-Glu-Val-Val-Glu-Glu-Ala-Glu-Asn

19. Medicament according to claim 18, characterised in that it contains the fragment VIII of the formula

7 8 9 10 11 12 13 14 15 16 17 18
Thr-Ser-Ser-Glu-Ile-Thr-Thr-Lys-Asp-Leu-Lys-Glu-
19 20 21 22 23 24 25 26 27 28
Lys-Lys-Glu-Val-Val-Glu-Glu-Ala-Glu-Asn

whose glutamic acid residues 10, 21, 24,25 and 27, whose asparagic acid residue 15 and/or whose asparagic residue 28 is present as an amide or an alkylamide and as a diamide or a dialkylamide, respectively, the alkyl groups having 1 to 6 carbon atoms.

20. Medicament according to claims 1 to 2, characterised in that it contains the fragment IX of the formula

1 2 3 4 5 6 7 8 9 10 11 12
Ser-Asp-Ala-Ala-Val-Asp-Thr-Ser-Ser-Glu-Ile-Thr

21. Medicament according to claim 20, characterised in that it contains the fragment IX of the formula

1 2 3 4 5 6 7 8 9 10 11 12
Ser-Asp-Ala-Ala-Val-Asp-Thr-Ser-Ser-Glu-Ile-Thr

whose glutamic acid residue 10 is present as an amide or an alkylamide with 1 to 6 carbon atoms in the alkyl group.

22. Medicament according to at least one of the preceding claims, characterised in that, in addition to the active substance or substances, it contains conventional pharmacologically acceptable binding agents, carrier materials and/or adjuvants suitable for the chosen administration route.

23. Thymosin$\alpha_1$-fragment I of the formula

1 2 3 4 5 6
Ser-Asp-Ala-Ala-Val-Asp

wherein the fragment N-terminal group can carry an acyl group with 1 to 6 carbon atoms, in particular an acetyl group or an acylglycine group, the acyl group of which has 1 to 6 carbon atoms, and also the pharmaceutically acceptable salts of this fragment.

24. Thymosin$\alpha_1$-fragment II of the formula

7 8 9 10 11 12
Thr-Ser-Ser-Glu-Ile-Thr

wherein the fragment N-terminal group can carry an acyl group with 1 to 6 carbon atoms, in particular an acetyl group or an acylglycine group, the acyl group of which has 1 to 6 carbon atoms, and the glutamic acid residue 10 may be present as an amide or an alkylamide with 1 to 6 carbon atoms in the alkyl group, and also the pharmaceutically acceptable salts of this fragment.

25. Thymosin$\alpha_1$-fragment III of the formula

13 14 15 16 17 18 19
Thr-Lys-Asp-Leu-Lys-Glu-Lys

wherein the fragment N-terminal group can carry an acyl group with 1 to 6 carbon atoms, in particular an acetyl group or an acylglycine group, the acyl group of which has 1 to 6 carbon atoms, and the asparagic acid residue 15 may be present as an amide or an alkylamide with 1 to 6 carbon atoms in the alkyl group, and also the pharmaceutically acceptable salts of this fragment.

26. Thymosin$\alpha_1$-fragment IV of the formula

20 21 22 23 24
Lys-Glu-Val-Val-Glu

wherein the fragment N-terminal group can carry an acyl group with 1 to 6 carbon atoms, in particular an acetyl group or an acylglycine group, the acyl group of which has 1 to 6 carbon atoms, and the glutamic acid residues 21 and 24 may be present as an amide or an alkylamide with 1 to 6 carbon atoms in the alkyl group, and also the pharmaceutically acceptable salts of this fragment.

27. Thymosin$\alpha_1$-fragment V of the formula

25 26 27 28
Glu-Ala-Glu-Asn

which N-terminal group carries an acyl group with 1 to 6 carbon atoms, in particular an acetyl group or an acylglycine group, the acyl group of which has 1 to 6 carbon atoms, and/or the glutamic acid residue 25 and/or 27 may be present as an amide or an alkylamide as well as the asparagine residue 28 as a

diamide or a dialkylamide with 1 to 6 carbon atoms in the alkyl group, and also the pharmaceutically acceptable salts of these derivatives.

28. Thymosin$\alpha_1$-fragment VI of the formula

20 21 22 23 24 25 26 27 28
Lys-Glu-Val-Val-Glu-Glu-Ala-Glu-Asn

wherein the fragment N-terminal group can carry an acyl group with 1 to 6 carbon atoms, in particular an acetyl group or an acyl glycine group with 1 to 6 carbon atoms in the acyl group, and the glutamic acid residues 21, 24, 25 and/or 27 as well as the asparagine residue 28 may be present as an amide or an alkylamide and as a diamide or a dialkylamide, respectively, with 1 to 6 carbon atoms in the alkyl groups, and also the pharmaceutically acceptable salts of this fragment.

29. Thymosin$\alpha_1$-fragment VII of the formula

13 14 15 16 17 18 19 20 21 22 23 24
Thr-Lys-Asp-Leu-Lys-Glu-Lys-Lys-Glu-Val-Val-Glu-
25 26 27 28
Glu-Ala-Glu-Asn

wherein the fragment N-terminal group can carry an acyl group with 1 to 6 carbon atoms, in particular an acetyl group or an acylglycine group, the acyl group of which has 1 to 6 carbon atoms, and the glutamic acid residues 21, 24, 25 and/or 27, the asparagic acid residue 15 as well as the asparagine residue 28 may be present as an amide or an alkylamide and as a diamide or as a dialkylamide respectively, with 1 to 6 carbon atoms in the alkyl groups, and also the pharmaceutically acceptable salts of this fragment.

30. Thymosin$\alpha_1$-fragment VIII of the formula

7 8 9 10 11 12 13 14 15 16 17 18
Thr-Ser-Ser-Glu-Ile-Thr-Thr-Lys-Asp-Leu-Lys-Glu-
19 20 21 22 23 24 25 26 27 28
Lys-Lys-Glu-Val-Val-Glu-Glu-Ala-Glu-Asn

wherein the fragment N-terminal group can carry an acyl group with 1 to 6 carbon atoms, in particular an acetyl group or an acylglycine group, the acyl group of which has 1 to 6 carbon atoms, and the glutamic acid residues 10, 21, 24, 25 and/or 27, the asparagic acid residue 15 as well as the asparagine residue 28 may be present as an amide or an alkylamide and as a diamide or as a dialkylamide, respectively, with 1 to 6 carbon atoms in the alkyl groups, and also the pharmaceutically acceptable salts of this fragment.

31. Thymosin$\alpha_1$-fragment IX of the formula

1 2 3 4 5 6 7 8 9 10 11 12
Ser-Asp-Ala-Ala-Val-Asp-Thr-Ser-Ser-Glu-Ile-Thr

wherein the fragment N-terminal group can carry an acyl group with 1 to 6 carbon atoms, in particular an acetyl group or acylglycine group, the acyl group of which has 1 to 6 carbon atoms, and the glutamic acid residue 10 may be present as an amide or as an alkylamide with 1 to 6 carbon atoms in the alkyl group, and also the pharmaceutically acceptable salts of this fragment.

32. Medicament according to claims 1 or 2, characterised in that it contains the fragment VIa, modified in position 24 and 25 by Gla-residues, of the formula

20 21 22 23 24 25 26 27 28
Lys-Glu-Val-Val-Gla-Gla-Ala-Glu-Asn (VIa)

33. Thymosin$\alpha_1$-fragment Ia of the formula

1 2 3 4 5 6
Lys-Asp-Ala-Ala-Val-Asp (Ia)

wherein the fragment N-terminal group can carry an acyl group with 1 to 6 carbon atoms, in particular an acetyl group or an acylglycine group, the acyl group of which has 1 to 6 carbon atoms, and also the pharmaceutically acceptable salts of this fragment.

34. Thymosin$\alpha_1$-fragment IVa of the formula

20 21 22 23 24
Lys-Glu-Val-Val-Gla (IVa)

wherein the fragment N-terminal group can carry an acyl group with 1 to 6 carbon atoms, in particular an acetyl group or an acylglycine group, the acyl group of which has 1 to 6 carbon atoms, and the glutamic acid residue 21 may be present as an amide or an alkylamide with 1 to 6 carbon atoms in the alkyl group, and also the pharmaceutically acceptable salts of this fragment.

35. Thymosin$\alpha_1$-fragment Va of the formula

25 26 27 28
Gla-Ala-Glu-Asn (Va)

wherein the fragment N-terminal group can carry an acyl group with 1 to 6 carbon atoms, in particular an acetyl group or an acylglycine group, the acyl group of which has 1 to 6 carbon atoms, and the glutamic acid residue 27 may be present as an amide or as an alkylamide and also the asparagine residue 28 may be present as a diamide or a dialkylamide with 1 to 6 carbon atoms in the alkyl group, as well as the pharmaceutically acceptable salts of this fragment.

36. Thymosin$\alpha_1$-fragment VIa of the formula

20 21 22 23 24 25 26 27 28
Lys-Glu-Val-Val-Gla-Gla-Ala-Glu-Asn (VIa)

wherein the fragment N-terminal group can carry an acyl group with 1 to 6 carbon atoms, in particular an acetyl group or an acylglycine group with 1 to 6 carbon atoms in the acyl group, and the glutamic acid residues 21 and/or 27 as well as the asparagine residue 28 may be present as an amide or an alkylamide and as a diamide or a dialkylamide, respectively, with 1 to 6 carbon atoms in the alkyl group, and also the pharmaceutically acceptable salts of this fragment.

**Revendications**

1. Médicament à effet immunostimulant, caractérisé en ce qu'il contient au moins un fragment de thymosine $\alpha_1$ et/ou au moins un dérivé d'un tel fragment à l'état libre ou à l'état de sel pharmacologiquement acceptable, à l'exception de ceux qui répondent à la formule générale:

X-Glu-Asn-OH

dans laquelle X représente:

H,
H-Ala-,
H-Glu-Ala-,
H-Glu-Glu-Ala-,
H-Val-Glu-Glu-Ala-
ou H-Val-Val-Glu-Glu-Ala-

et de leurs sels physiologiquement acceptables.

2. Médicament selon la revendication 1, caractérisé en ce qu'il contient en tant que dérivé du fragment de thymosine $\alpha_1$, un fragment de thymosine $\alpha_1$, dont l'azote terminal est à l'état libre ou porte un groupe acyle en $C_1$-$C_6$, de préférence un groupe acétyle ou un groupe acylglycine dont le groupe acyle présente de 1 à 6 atomes de carbone.

3. Médicament selon l'une quelconque des revendications 1 ou 2, caractérisé en ce qu'il contient le fragment I:

1 2 3 4 5 6
Ser-Asp-Ala-Ala-Val-Asp

4. Médicament selon l'une quelconque des revendications 1 ou 2, caractérisé en ce qu'il contient le fragment modifié en position 1 par un reste de lysine, de formule:

1 2 3 4 5 6
Lys-Asp-Ala-Ala-Val-Asp

5. Médicament selon l'une quelconque des revendications 1 ou 2, caractérisé en ce qu'il contient le fragment II de formule:

7 8 9 10 11 12
Thr-Ser-Ser-Glu-Ile-Thr

6. Médicament selon la revendication 5, caractérisé en ce qu'il contient le fragment II:

7 8 9 10 11 12
Thr-Ser-Ser-Glu-Ile-Thr

dont le reste d'acide glutamique 10 est à l'état d'amide ou d'alkylamide contenant de 1 à 6 atomes de carbone dans le groupe alkyle.

7. Médicament selon l'une quelconque des revendications 1 ou 2, caractérisé en ce qu'il contient le fragment III de formule:

13 14 15 16 17 18 19
Thr-Lys-Asp-Leu-Lys-Glu-Lys

8. Médicament selon la revendication 7, caractérisé en ce qu'il contient le fragment III de formule:

13 14 15 16 17 18 19
Thr-Lys-Asp-Leu-Lys-Glu-Lys

dont le reste d'acide aspartique 15 est à l'état d'amide ou d'alkylamide contenant de 1 à 6 atomes de carbone dans le groupe alkyle.

9. Médicament selon l'une quelconque des revendications 1 ou 2, caractérisé en ce qu'il contient le fragment IV de formule:

20 21 22 23 24
Lys-Glu-Val-Val-Glu

10. Médicament selon l'une quelconque des revendications 1 ou 2, caractérisé en ce qu'il contient le fragment IVa modifié en position 24 par un reste d'acide $\gamma$-carboxyglutamique-(Gla) de formule:

20 21 22 23 24
Lys-Glu-Val-Val-Gla

11. Médicament selon la revendication 10, caractérisé en ce qu'il contient le fragment IV de formule:

20 21 22 23 24
Lys-Glu-Val-Val-Glu

dans lequel le reste d'acide glutamique 21 et/ou 24 est à l'état d'amide ou d'alkylamide contenant de 1 à 6 atomes de carbone dans le groupe alkyle.

12. Médicament selon l'une quelconque des revendications 1 ou 2, caractérisé en ce qu'il contient le fragment Va modifié en position 25 par le reste Gla, de formule:

25 26 27 28
Gla-Ala-Glu-Asn

13. Médicament selon la revendication 12, caractérisé en ce qu'il contient le fragment V de formule:

25 26 27 28
Gla-Ala-Glu-Asn

dont le reste d'acide glutamique 25 et/ou 27 ainsi que le reste d'asparagine 28 est à l'état respectivement d'amide ou d'alkylamide et de diamide ou de dialkylamide, les groupes alkyle contenant de 1 à 6 atomes de carbone.

14. Médicament selon l'une quelconque des revendications 1 ou 2, caractérisé en ce qu'il contient le fragment VI de formule:

20 21 22 23 24 25 26 27 28
Lys-Glu-Val-Val-Glu-Glu-Ala-Glu-Asn

15. Médicament selon la revendication 14, caractérisé en ce qu'il contient le fragment VI de formule:

20 21 22 23 24 25 26 27 28
Lys-Glu-Val-Val-Glu-Glu-Ala-Glu-Asn

dont les restes d'acide glutamique 21 et 25 et/ou le reste d'asparagine 28 sont respectivement à l'état d'amide ou d'alkylamide et de diamide ou dialkylamide, les groupes alkyle contenant de 1 à 6 atomes de carbone.

16. Médicament selon l'une quelconque des revendications 1 ou 2, caractérisé en ce qu'il contient le fragment VII de formule:

13 14 15 16 17 18 19 20 21 22 23 24
Thr-Lys-Asp-Leu-Lys-Glu-Lys-Lys-Glu-Val-Val-Glu-
25 26 27 28
Glu-Ala-Glu-Asn

17. Médicament selon la revendication 16, caractérisé en ce qu'il contient le fragment VII de formule:

13 14 15 16 17 18 19 20 21 22 23 24
Thr-Lys-Asp-Leu-Lys-Glu-Lys-Lys-Glu-Val-Val-Glu-
25 26 27 28
Glu-Ala-Glu-Asn

dont le reste d'acide aspartique 15 et/ou les restes d'acide glutamique 21, 24, 25 et/ou 27 et le reste d'asparagine 28 sont à l'état respectivement d'amide ou d'alkylamide et de diamide ou de dialkylamide contenant de 1 à 6 atomes de carbone dans le groupe alkyle.

18. Médicament selon l'une quelconque des revendications 1 ou 2, caractérisé en ce qu'il contient le fragment VIII:

7 8 9 10 11 12 13 14 15 16 17 18
Thr-Ser-Ser-Glu-Ile-Thr-Thr-Lys-Asp-Leu-Lys-Glu-
19 20 21 22 23 24 25 26 27 28
Lys-Lys-Glu-Val-Val-Glu-Glu-Ala-Glu-Asn

19. Médicament selon la revendication 18, caractérisé en ce qu'il contient le fragment VIII:

7 8 9 10 11 12 13 14 15 16 17 18
Thr-Ser-Ser-Glu-Ile-Thr-Thr-Lys-Asp-Leu-Lys-Glu-
19 20 21 22 23 24 25 26 27 28
Lys-Lys-Glu-Val-Val-Glu-Glu-Ala-Glu-Asn

dont les restes d'acide glutamique 10, 21, 24, 25 et 27, le reste d'acide aspartique 15 et/ou le reste d'asparagine 28 sont respectivement à l'état d'amide ou d'alkylamide et de diamide ou de dialkylamide, les groupes alkyle contenant de 1 à 6 atomes de carbone.

20. Médicament selon l'une quelconque des revendications 1 ou 2, caractérisé en ce qu'il contient le fragment IX:

1 2 3 4 5 6 7 8 9 10 11 12
Ser-Asp-Ala-Ala-Val-Asp-Thr-Ser-Ser-Glu-Ile-Thr

21. Médicament selon la revendication 20, caractérisé en ce qu'il contient le fragment IX de formule:

1 2 3 4 5 6 7 8 9 10 11 12
Ser-Asp-Ala-Ala-Val-Asp-Thr-Ser-Ser-Glu-Ile-Thr

dont le reste d'acide glutamique 10 est à l'état d'amide ou d'alkylamide contenant de 1 à 6 atomes de carbone dans le groupe alkyle.

22. Médicament selon au moins l'une des revendications qui précèdent, caractérisé en ce qu'il contient, avec la ou les substances actives, des liants, véhicules et/ou produits auxiliaires usuels, pharmaceutiquement acceptables et appropriés au mode d'administration choisi.

23. Fragment I de la thymosine $\alpha_1$, de formule:

1 2 3 4 5 6
Lys-Asp-Ala-Ala-Val-Asp

dans lequel l'azote terminal du fragment peut porter un groupe acyle en $C_1$-$C_6$, en particulier un groupe acétyle ou un groupe acylglycine dont le groupe acyle contient de 1 à 6 atomes de carbone, ainsi que les sels pharmaceutiquement acceptables de ce fragment.

24. Fragment II de la thymosine $\alpha_1$, de formule:

7 8 9 10 11 12
Thr-Ser-Ser-Glu-Ile-Thr

dans lequel l'azote terminal du fragment peut porter un groupe acyle en $C_1$-$C_6$, en particulier un groupe acétyle ou un groupe acylglycine dont le groupe acyle contient de 1 à 6 atomes de carbone, et le reste d'acide glutamique 10 peut être à l'état d'amide ou d'alkylamide contenant de 1 à 6 atomes de carbone dans le groupe alkyle, ainsi que les sels pharmaceutiquement acceptables de ce fragment.

25. Fragment III de la thymosine $\alpha_1$, de formule:

13 14 15 16 17 18 19
Thr-Lys-Asp-Leu-Lys-Glu-Lys

dans lequel l'azote terminal du fragment peut porter un groupe acyle de 1 à 6 atomes de carbone, en particulier un groupe acétyle ou un groupe acylglycine dont le groupe acyle contient de 1 à 6 atomes de carbone, et le reste d'acide aspartique 15 peut être à l'état d'amide ou d'alkylamide contenant de 1 à 6 atomes de carbone dans le groupe alkyle, ainsi que les sels pharmaceutiquement acceptables de ce fragment.

26. Fragment IV de la thymosine $\alpha_1$, de formule:

20 21 22 23 24
Lys-Glu-Val-Val-Glu

dans lequel l'azote terminal du fragment peut porter un groupe acyle en $C_1$-$C_6$, en particulier un groupe acétyle ou un groupe acylglycine dont le groupe acyle contient de 1 à 6 atomes de carbone, et le reste d'acide glutamique 21 et 24 peut être à l'état d'amide ou d'alkylamide contenant de 1 à 6 atomes de carbone dans le groupe alkyle, ainsi que les sels pharmaceutiquement acceptables de ce fragment.

27. Fragment V de la thymosine $\alpha_1$, de formule:

25 26 27 28
Glu-Ala-Glu-Asn

portant sur l'azote terminal un groupe acyle en

$C_1$-$C_6$, en particulier un groupe acétyle ou un groupe acylglycine dont le groupe acyle contient de 1 à 6 atomes de carbone, et/ou le reste d'acide glutamique 25 et/ou 27 peut être à l'état d'amide ou d'alkylamide, et le reste d'asparagine 28 peut être à l'état de diamide ou de dialkylamide contenant de 1 à 6 atomes de carbone dans le groupe alkyle, ainsi que les sels pharmaceutiquement acceptables de ces dérivés.

28. Fragment VI de la thymosine $\alpha_1$, de formule:

20 21 22 23 24 25 26 27 28
Lys-Glu-Val-Val-Glu-Glu-Ala-Glu-Asn

dans lequel le fragment peut porter sur l'azote terminal un groupe acyle en $C_1$-$C_6$, en particulier un groupe acétyle ou un groupe acylglycine contenant de 1 à 6 atomes de carbone dans le groupe acyle, et les restes d'acide glutamique 21, 24, 25 et/ou 27 ainsi que le reste d'asparagine 28 peuvent exister à l'état respectivement d'amide ou d'alkylamide et de diamide ou de dialkylamide contenant de 1 à 6 atomes de carbone dans les groupes alkyle, ainsi que les sels pharmaceutiquement acceptables de ce fragment.

29. Fragment VII de la thymosine $\alpha_1$, de formule:

13 14 15 16 17 18 19 20 21 22 23 24
Thr-Lys-Asp-Leu-Lys-Glu-Lys-Lys-Glu-Val-Val-Glu-
25 26 27 28
Glu-Ala-Glu-Asn

dans lequel le fragment peut porter sur l'azote terminal un groupe acyle en $C_1$-$C_6$, en particulier un groupe acétyle ou un groupe acylglycine dont le groupe acyle contient de 1 à 6 atomes de carbone, et les restes d'acide glutamique 21, 24, 25 et/ou 27, le reste d'acide aspartique 15 ainsi que le reste d'asparagine 28 peut être à l'état respectivement d'amide ou d'alkylamide et de diamide ou dialkylamide contenant de 1 à 6 atomes de carbone dans les groupes alkyle, ainsi que les sels pharmaceutiquement acceptables de ce fragment.

30. Fragment VIII de la thymosine $\alpha_1$, de formule:

7 8 9 10 11 12 13 14 15 16 17 18
Thr-Ser-Ser-Glu-Ile-Thr-Thr-Lys-Asp-Leu-Lys-Glu-
19 20 21 22 23 24 25 26 27 28
Lys-Lys-Glu-Val-Val-Glu-Glu-Ala-Glu-Asn

dans lequel le fragment peut porter sur l'azote terminal un groupe acyle en $C_1$-$C_6$, en particulier un groupe acétyle ou un groupe acylglycine dont le groupe acyle est en $C_1$-$C_6$, et les restes d'acide glutamique 10, 21, 24, 25 et/ou 27, le reste d'acide aspartique 15 et le reste d'asparagine 28 peuvent être à l'état respectivement d'amide ou d'alkylamide et de diamide ou dialkylamide contenant de 1 à 6 atomes de carbone dans les groupes alkyle, ainsi que les sels pharmaceutiquement acceptables de ce fragment.

31. Fragment IX de la thymosine $\alpha_1$, de formule:

1 2 3 4 5 6 7 8 9 10 11 12
Ser-Asp-Ala-Ala-Val-Asp-Thr-Ser-Ser-Glu-Ile-Thr

dans lequel le fragment peut porter sur l'azote terminal un groupe acyle en $C_1$-$C_6$, en particulier un groupe acétyle ou un groupe acylglycine dont le groupe acyle est en $C_1$-$C_6$, et le reste d'acide glutamique 10 peut être à l'état d'amide ou d'alkylamide contenant de 1 à 6 atomes de carbone dans le groupe alkyle, ainsi que les sels pharmaceutiquement acceptables de ce fragment.

32. Médicament selon l'une quelconque des revendications 1 ou 2, caractérisé en ce qu'il contient le fragment VIa, modifié en positions 24 et 25 par des restes Gla, de formule:

20 21 22 23 24 25 26 27 28
Lys-Glu-Val-Val-Gla-Gla-Ala-Glu-Asn (VIa)

33. Fragment Ia de la thymosine-$\alpha_1$, de formule:

1 2 3 4 5 6
Lys-Asp-Ala-Ala-Val-Asp (Ia)

dans lequel le fragment peut porter sur l'azote terminal un groupe acyle en $C_1$-$C_6$, en particulier un groupe acétyle ou un groupe acylglycine dont le groupe acyle contient de 1 à 6 atomes de carbone, ainsi que les sels pharmaceutiquement acceptables de ce fragment.

34. Fragment IVa de la thymosine $\alpha_1$, de formule:

20 21 22 23 24
Lys-Glu-Val-Val-Gla (IVa)

dans lequel le fragment peut porter sur l'azote terminal un groupe acyle en $C_1$-$C_6$, en particulier un groupe acétyle ou un groupe acylglycine dont le groupe acyle contient de 1 à 6 atomes de carbone, et le reste d'acide glutamique 21 pouvant être à l'état d'amide ou d'alkylamide contenant de 1 à 6 atomes de carbone dans le groupe alkyle, ainsi que les sels pharmaceutiquement acceptables de ce fragment.

35. Fragment Va de la thymosine $\alpha_1$, de formule:

25 26 27 28
Gla-Ala-Glu-Asn (Va)

dans lequel le fragment peut porter sur l'azote terminal un groupe acyle en $C_1$-$C_6$, en particulier un groupe acétyle ou un groupe acylglycine dont le groupe acyle contient de 1 à 6 atomes de carbone, et le reste d'acide glutamique 27 peut être à l'état d'amide ou d'alkylamide et le reste d'asparagine 28 à l'état de diamide ou de dialkylamide contenant de 1 à 6 atomes de carbone dans le groupe alkyle, ainsi que les sels pharmaceutiquement acceptables de ce fragment.

36. Fragment VIa de la thymosine $\alpha_1$, de formule:

20 21 22 23 24 25 26 27 28
Lys-Glu-Val-Val-Gla-Gla-Ala-Glu-Asn (VIa)

dans lequel le fragment peut porter sur l'azote terminal un groupe acyle en $C_1$-$C_6$, en particulier un groupe acétyle ou un groupe acylglycine contenant de 1 à 6 atomes de carbone dans le groupe acyle, et les restes d'acide glutamique 21 et/ou 27 ainsi que le reste d'asparagine 28 peuvent être à l'état respectivement d'amide ou d'alkylamide et de diamide ou de dialkylamide contenant de 1 à 6 atomes de carbone dans le groupe alkyle, ainsi que les sels pharmaceutiquement acceptables de ce fragment.

FIG.1

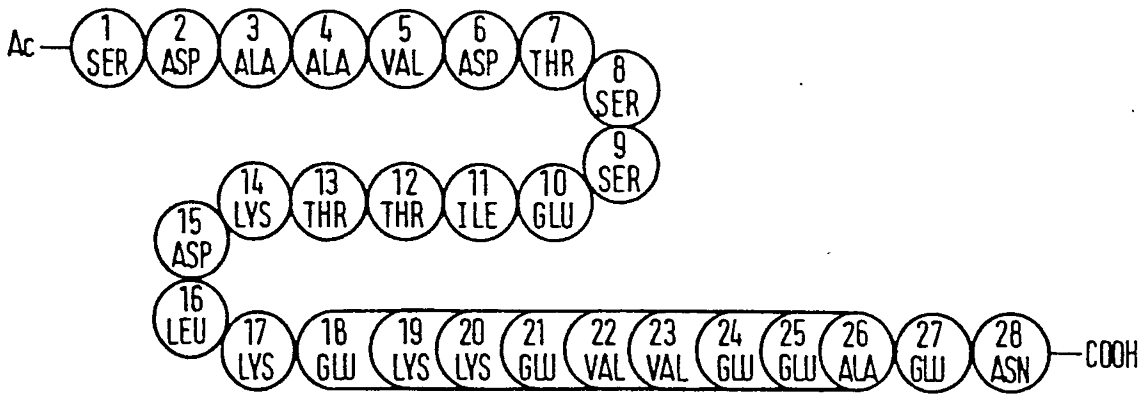


Thymosin $\alpha_1$

FIG. 2

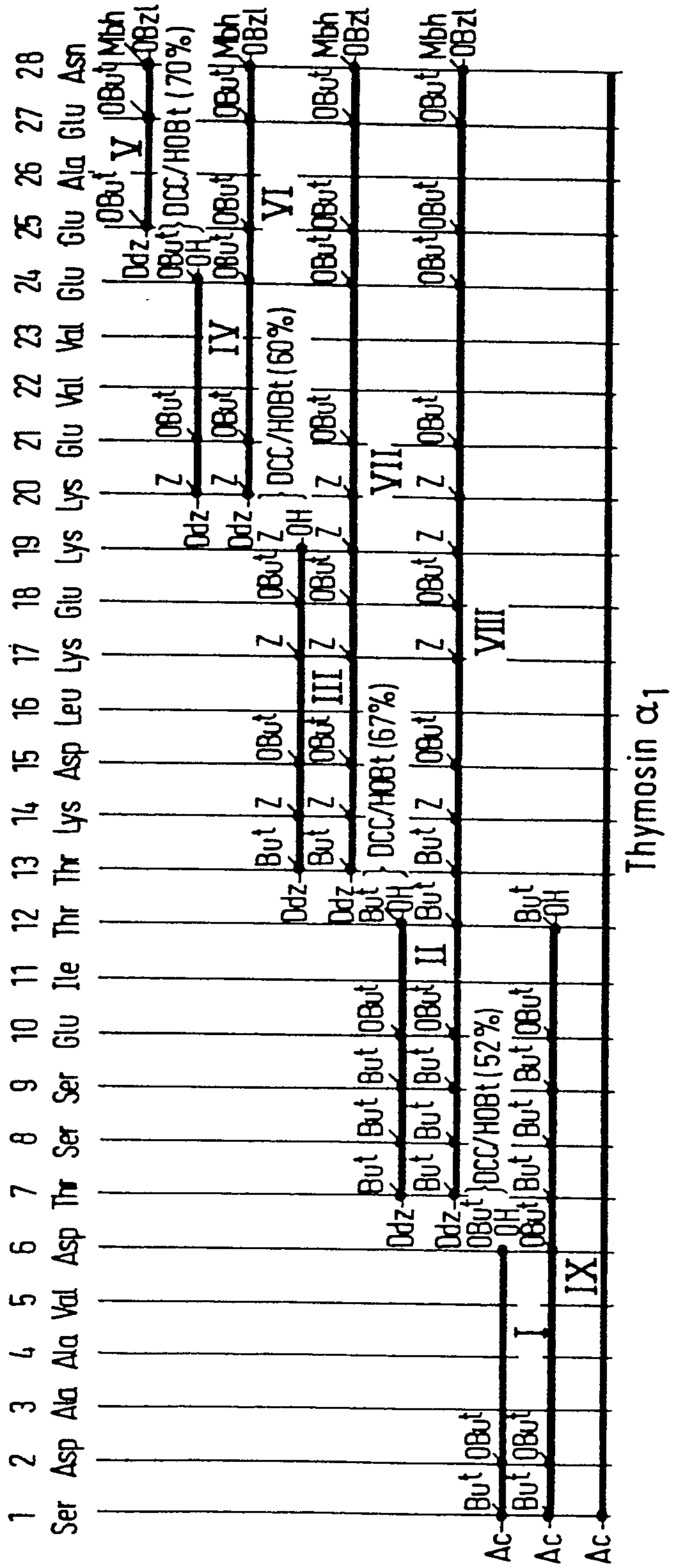
1 Ser
2 Asp
3 Ala
4 Ala
5 Val
6 Asp
7 Thr
8 Ser
9 Ser
10 Glu
11 Ile
12 Thr
13 Thr
14 Lys
15 Asp
16 Leu
17 Lys
18 Glu
19 Lys
20 Lys
21 Glu
22 Val
23 Val
24 Glu
25 Glu
26 Ala
27 Glu
28 Asn
DCC/HOBt (70%)
DCC/HOBt (60%)
DCC/HOBt (67%)
DCC/HOBt (52%)
I
II
III
IV
V
VI
VII
VIII
IX
Thymosin α1

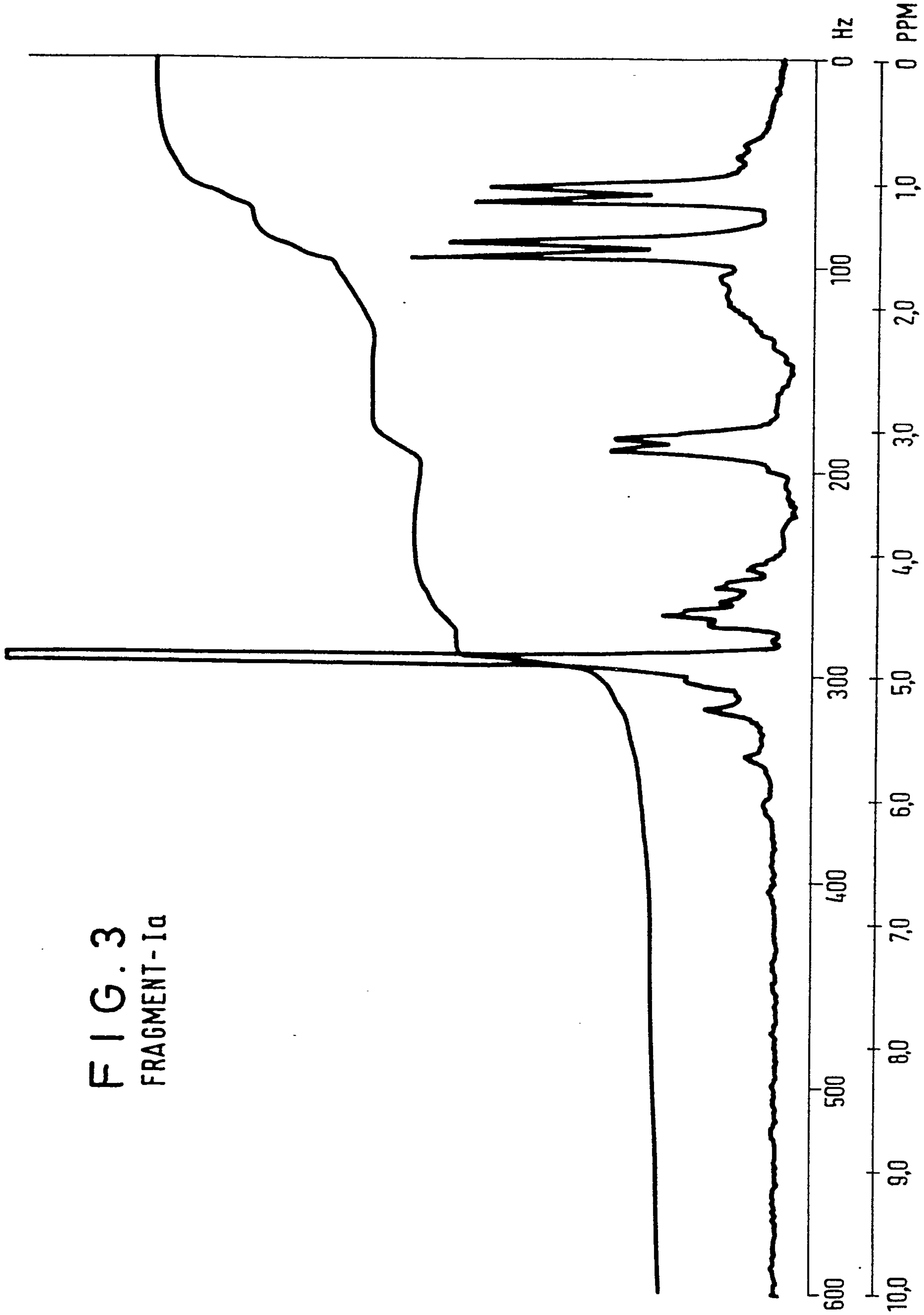


F I G. 3
FRAGMENT-Ia

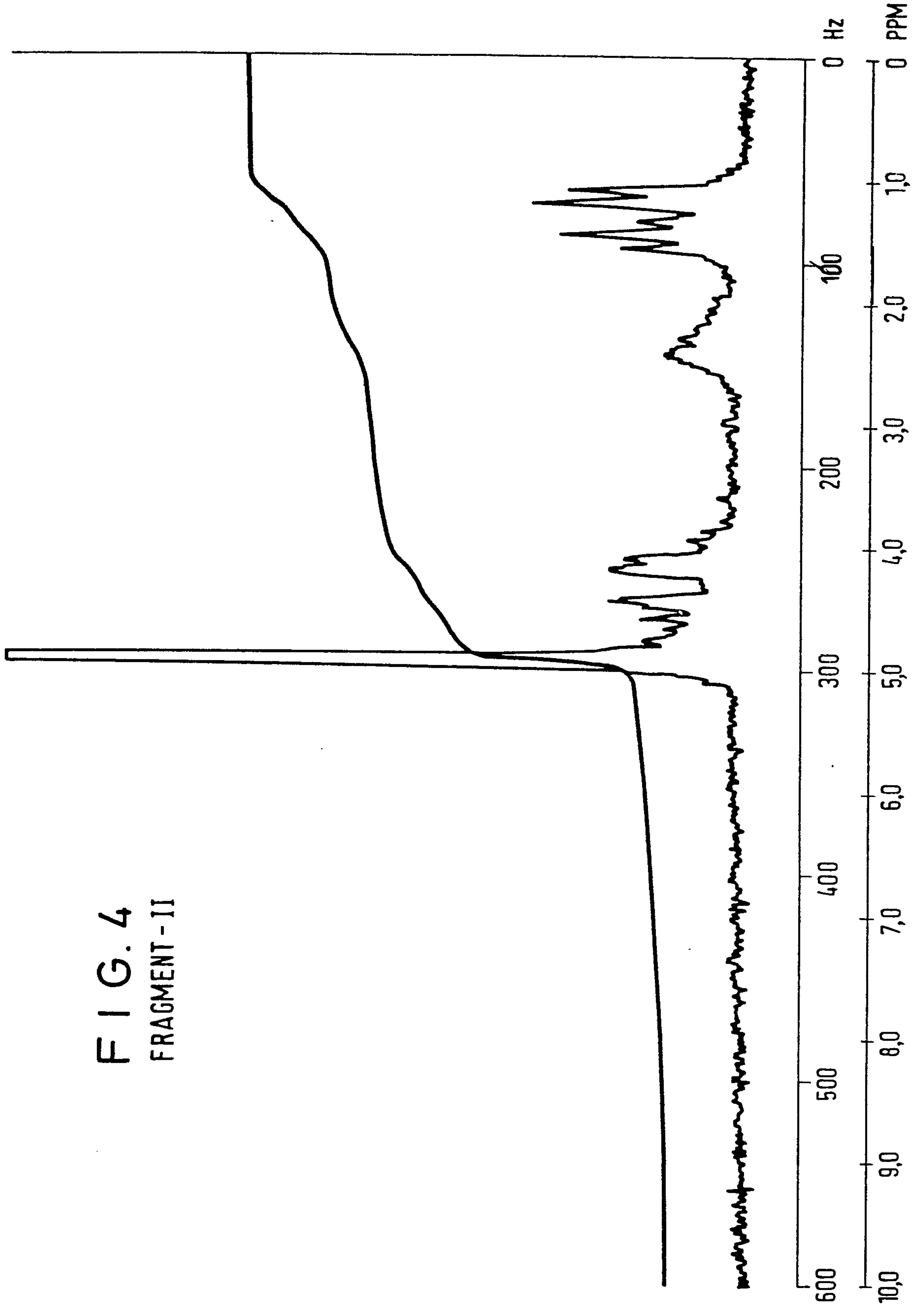


FIG. 4
FRAGMENT-II

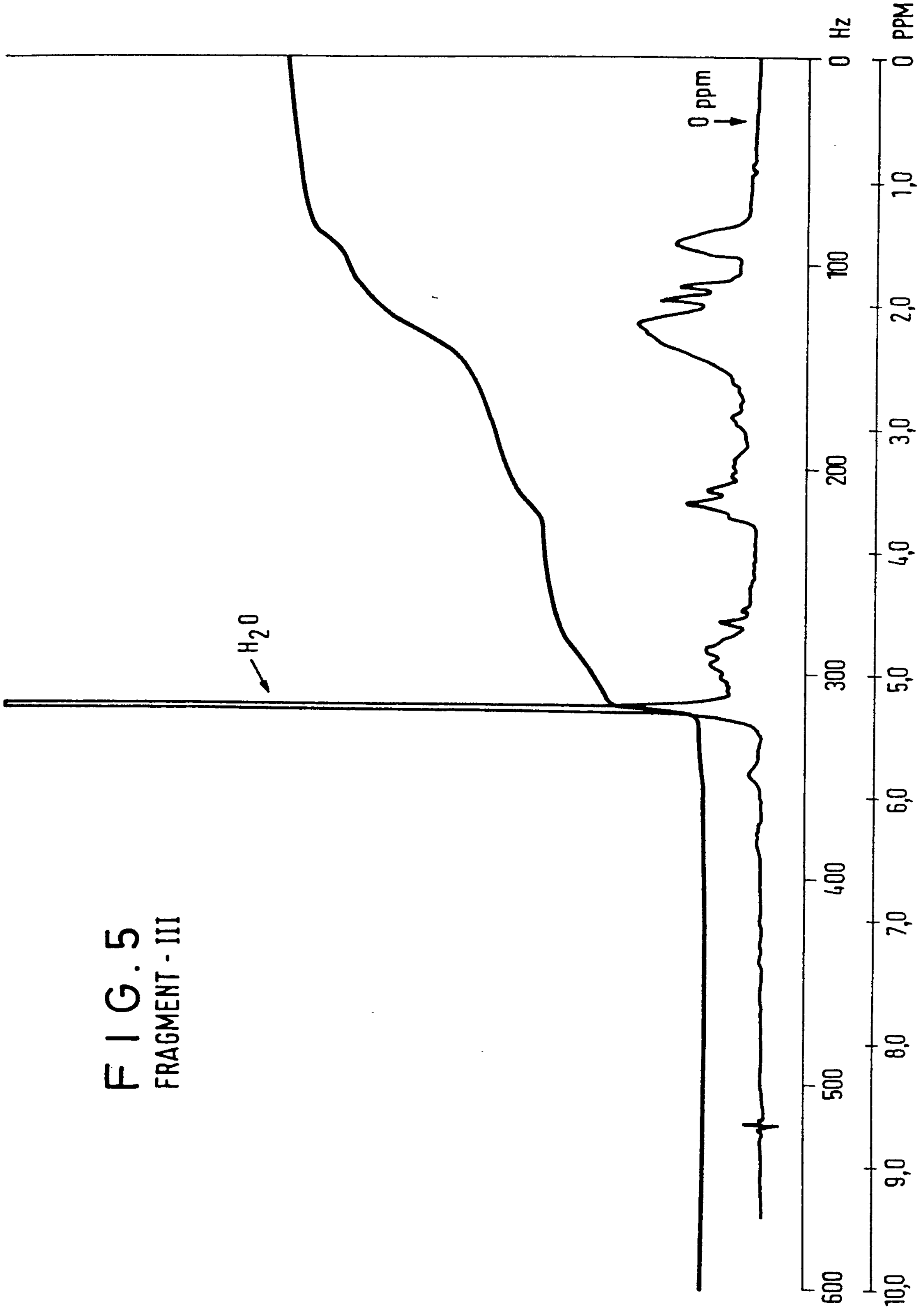


FIG. 5
FRAGMENT - III

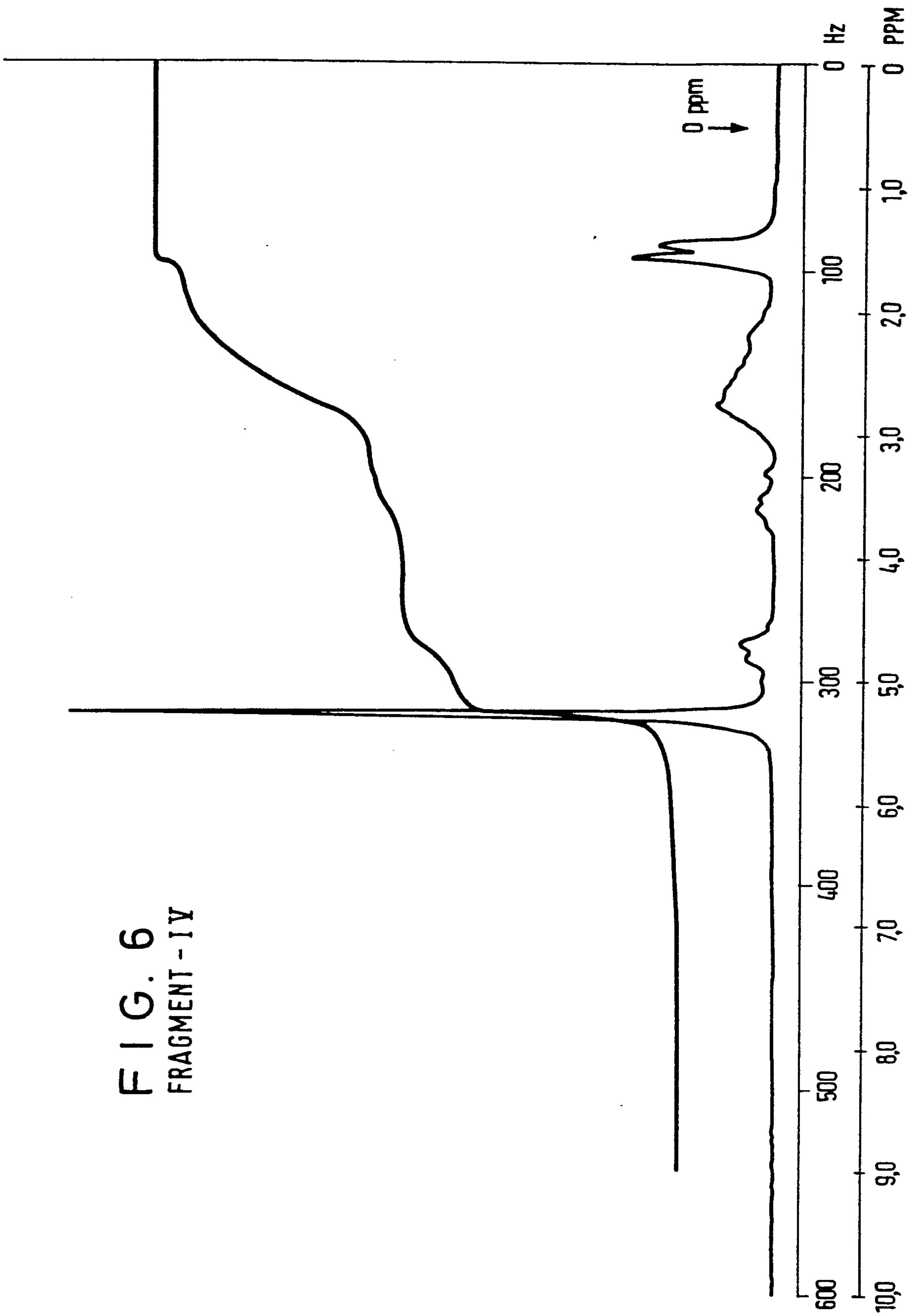


F I G. 6
FRAGMENT - IV

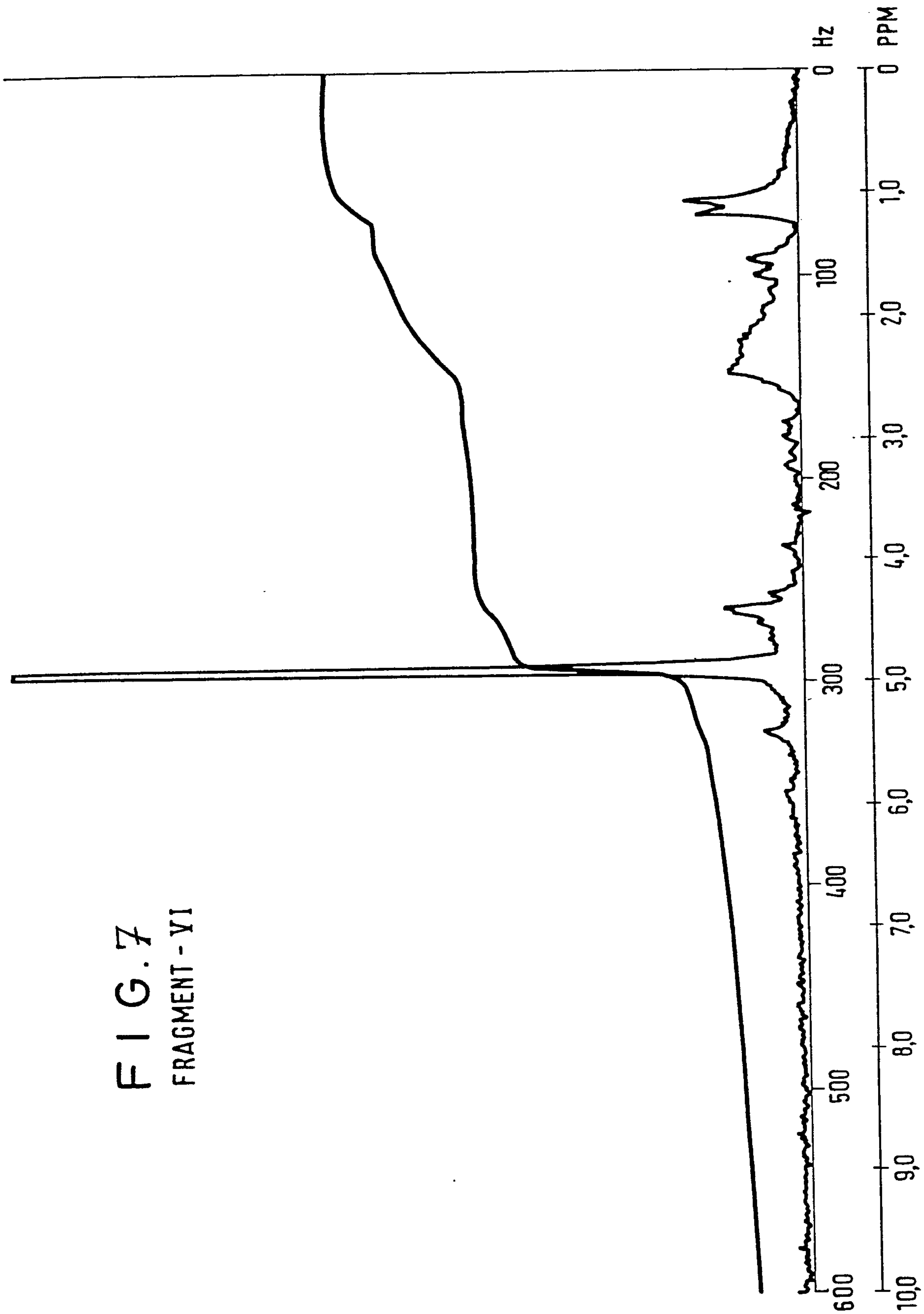


F I G. 7
FRAGMENT - VI

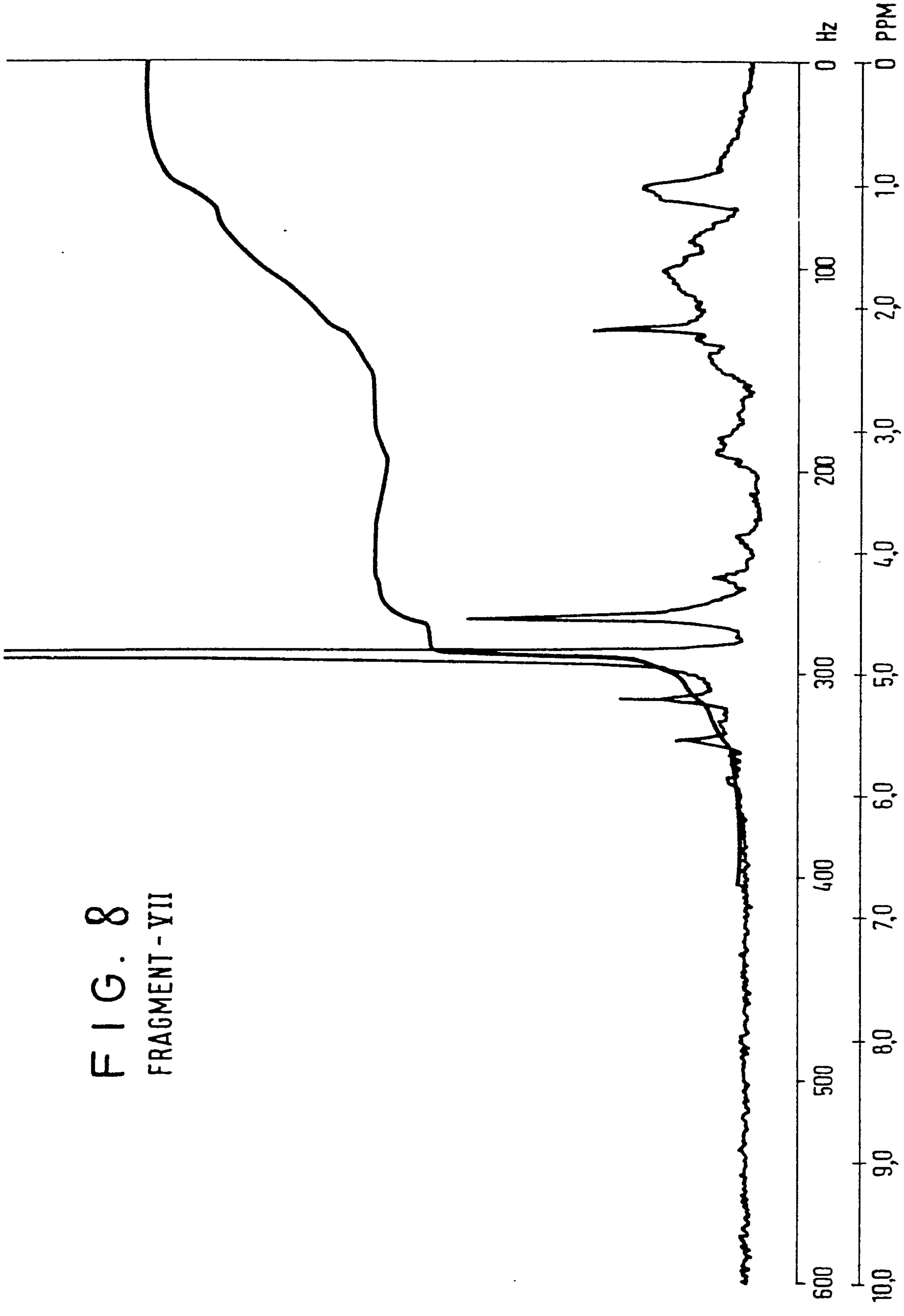


FIG. 8
FRAGMENT-VII

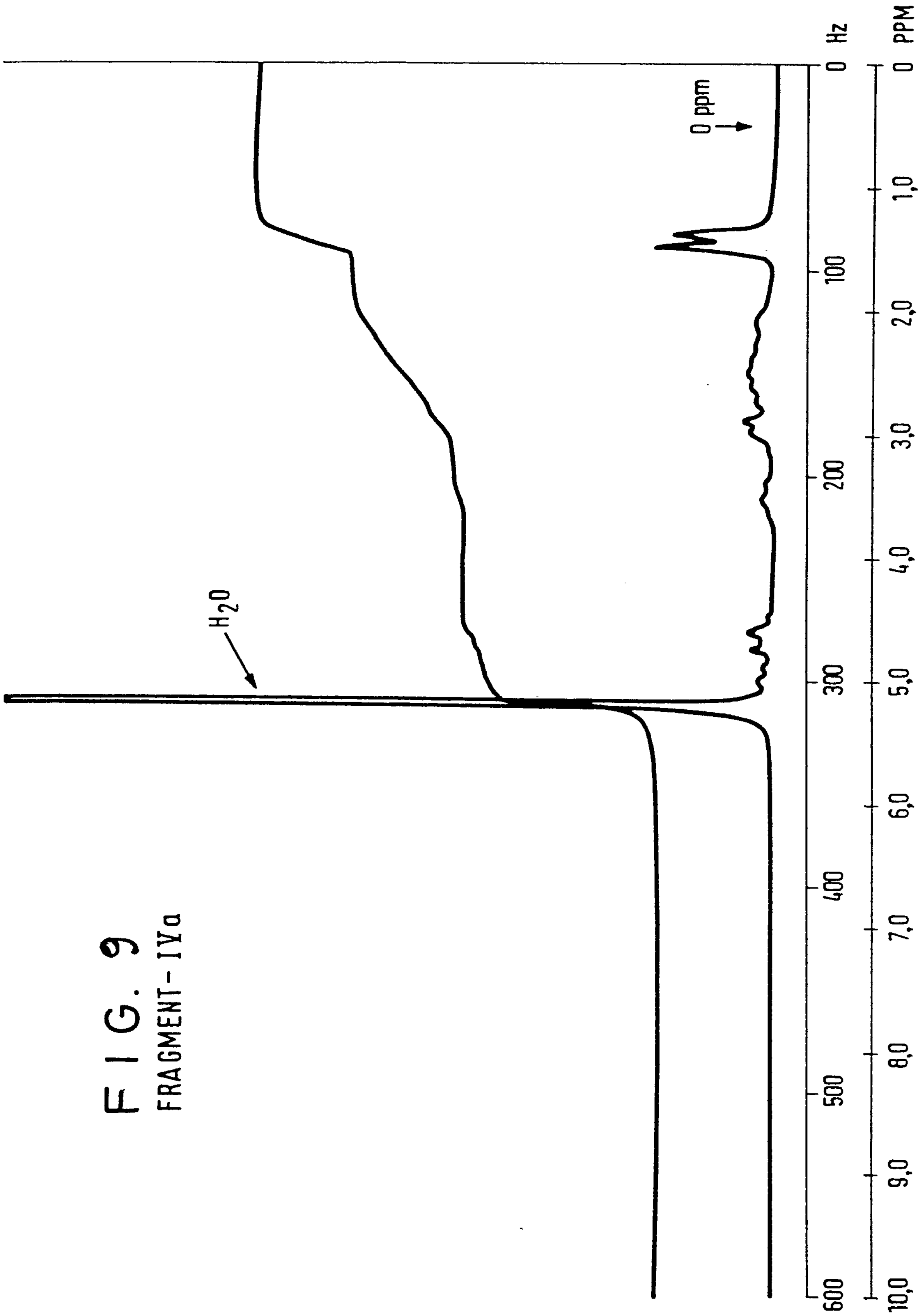


FIG. 9
FRAGMENT-IVa